# EUROPEAN PATENT SPECIFICATION

(11) **EP 0 733 066 B1**
(45) Date of publication and mention of the grant of the patent: **19.11.2003**
(21) Application number: 95905334.9
(22) Date of filing: 07.12.1994
(51) Int. Cl.: C07K 14/52, C07K 14/525, C07K 14/53, C07K 14/535, C07K 14/54, C07K 14/555, C07K 16/00, C07K 16/30, A61K 38/17, A61K 38/38, A61K 47/48

(54) **PRETARGETING METHODS AND COMPOUNDS**
PRETARGETING VERFAHREN UND MITTELN
PROCEDES ET COMPOSES DE PRECIBLAGE

(30) Priority: 07.12.1993 US 163188
(43) Date of publication of application: 25.09.1996
(60) Divisional application: 03008765.4
(73) Proprietor: NeoRx Corporation, Seattle Washington 98119 (US)
(72) Inventor: THEODORE, Louis J., Lynnwood, WA 98037 (US); MEYER, Damon L., Bellevue, WA 98006 (US); MALLETT, Robert W., Seattle, WA 98133 (US); KASINA, Sudhakar, Kirkland, WA 98034 (US); RENO, John M., Brier, WA 98036 (US); AXWORTHY, Donald B., Brier, WA 98036 (US); GUSTAVSON, Linda M., Seattle, WA 98155 (US)
(74) Representative: Jones, Helen Marjorie Meredith
(86) International application number: US9414174
(87) International publication number: WO95015979

(56) References cited:
- EP-A- 0 251 494
- EP-A- 0 496 074
- WO-A-89/10140
- WO-A-93/25240
- PAGANELLI G ET AL: "MONOCLONAL ANTIBODY PRETARGETING TECHNIQUES FOR TUMOUR LOCALIZATIONTHE AVIDIN-BIOTIN SYSTEM" NUCLEAR MEDICINE COMMUNICATIONS, vol. 12, no. 3, 1 March 1991, pages 211-234, XP000373577
- GOODWIN D.A.: "New Methods for Localizing Infection: a Role for Avidin-Biotin ? " THE JOURNAL OF NUCLEAR MEDICINE, vol. 33, no. 10, 1992, pages 1816-1818, XP002094397
- GALLI ET AL.: "A Radiopharmaceutical for the Study of the Liver: Tc-DTPA-Asialo-Orosomucoid" THE JOURNAL OF NUCLEAR MEDICINE AND ALLIED SCIENCES, vol. 32, no. 2, 1988, pages 117-126, XP002094398
- SANDERSON J.A. ET AL.: "Preparation and Characterization of Biotin Conjugates of Anti-Pan-Carcinoma NR-LU-10 monoclonal Antibody for a Three Step Radioimmunotherapy. Page 880 (Abstract Number 233)" THE JOURNAL OF NUCLEAR MEDICINE; PROCEEDINGS OF THE 39TH ANNUAL MEETING, vol. 33, 13 May 1992, page 880 XP002094399
- AXWORTHY D.B.: "Antibody Pretargeting for Radioimmunotherapy: a Three Step Approach in Tumored Nude Mice" THE JOURNAL OF NUCLEAR MEDICINE; PROCEEDINGS OF THE 39TH ANNUAL MEETING, vol. 33, 13 May 1992, XP002094400
- PROC. NATL. ACAD. SCI. USA, Vol. 90, issued 01 October 1993, F.J. BURROWS et al., "Eradication of Large Solid Tumors in Mice with an Immunotoxin Directed Against Tumor Vasculature", pages 8996-9000.
- J. NATL. CANC. INST., Volume 85, No. 6, issued 13 March 1993, H.I. PASS, "Photodynamic Therapy in Oncology: Mechanisms and Clinical Use", pages 443-456.
- J. BIOL. CHEM., Vol. 252, No. 11, issued 10 June 1977, A. ABUCHOWSKI et al., "Effect of Covalent Attachment of Polyethylene Glycol on Immunogenicity and Circulating Life of Bovine Liver Catalase", pages 3582-3586.
- IMMUNOLOGY LETTERS, Vol. 15, issued 1987, I. WILKINSON et al., "Tolerogenic Polyethylene Glycol Derivatives of Xenogeneic Monoclonal Immunoglobulins", pages 17-22.

## Description

### Technical Field

The present invention relates to methods, compounds, compositions and kits useful for delivering to a target site a targeting moiety that is conjugated to one member of a ligand/anti-ligand pair. After localization and optional clearance of the targeting moiety conjugate, binding of a diagnostic or therapeutic agent conjugate at the target site occurs.

### Background of the Invention

Conventional cancer therapy is plagued by two problems. The generally attainable targeting ratio (ratio of administered dose localizing to tumor versus administered dose circulating in blood or ratio of administered dose localizing to tumor versus administered dose migrating to bone marrow) is low. Also, the absolute dose of radiation or therapeutic agent delivered to the tumor is insufficient in many cases to elicit a significant tumor response. Improvement in targeting ratio or absolute dose to tumor is sought.

### Summary of the Invention

The present invention is directed to diagnostic and therapeutic pretargeting methods, moieties useful therein and methods of making those moieties. Such pretargeting methods are characterized by an improved targeting ratio or increased absolute dose to the target cell sites in comparison to conventional cancer therapy.

The present invention provides for effective delivery of cytotoxic active agents such as toxins, bacterial toxins and fungal metabolites, including highly toxic moieties such as palytoxins. The decoupling of the pharmacokinetics of the targeting moiety (generally slow) and the active agent (generally rapid for low molecular weight active agents and generally somewhat slower for higher molecular weight active agents when administered alone) and the high affinity binding of ligand-anti-ligand pairs provide for this improvement. When the active agents or active agent-ligand or active agent-anti-ligand conjugates to be administered are not themselves generally rapidly cleared (preferably via the renal pathway), conjugates containing such active agents are constructed to impart relatively rapid, and preferably renal, clearance thereto or lower, therapeutically effective, doses of active agent are administered. Thus, the protocol recipient's non-target tissue does not suffer prolonged exposure to the active agent.

The present invention provides two-step pretargeting methods, employing the steps set forth below:
administering to the recipient a first conjugate including an antibody targeting moiety of a first antibody species having a first pattern of cross-reactivity and a member of a ligand-anti-ligand binding pair; and
administering to the recipient one or more additional targeting conjugates, each such conjugate including an antibody targeting moiety of a different species from the species of the first conjugate and each other having a substantially non-overlapping pattern of cross reactivity from that of other additional targeting conjugates and from each other and from the first pattern of cross-reactivity and the member of the ligand-anti-ligand pair bound to the first conjugate.

In the practice of these aspects of the present invention, target site accretion of active agent conjugate receptor (i.e., the ligand or anti-ligand conjugated to the first antibody species and additional targeting antibody species) is improved, because each antibody species recognizes a different epitope associated with the target site. This alternative epitope approach provides a target site that is more densely populated with the anti-ligand or ligand antigen to which the subsequently administered active agent-containing conjugate may bind via high affinity ligand-anti-ligand interactions. This increased target site antigen density facilitates increased active agent accretion thereto.

The present invention also provides pretargeting photodynamic therapy protocols as set forth below.

The two-step approach involves:
administering to the recipient a first conjugate comprising a targeting moiety and a member of a ligand-anti-ligand binding pair, wherein the first conjugate localizes to a target site;
optionally administering to the recipient a clearing agent capable of directing the clearance of circulating conjugate from the recipient or optionally treating the recipient with a clearing device or an alternative clearing procedure to substantially remove circulating conjugate from the recipient; and
administering to the recipient a second conjugate comprising a photosensitizing agent and a ligand/anti-ligand binding pair member, wherein the second conjugate binding pair member is complementary to that of the first conjugate and wherein the photosensitizing agent or the second conjugate is chemically modified to induce rapid and, preferably, renal clearance thereof from the recipient.

One alternative to the optional clearance step set forth above is simply to allow an amount of time to pass that is sufficient to permit the recipient's native clearance mechanisms to substantially remove circulating first conjugate.

While the pretargeting methods of the present invention may be conducted despite the presence of recipient endogenous biotin, the present invention also provides methods of decreasing the endogenous biotin level or the impact thereof. One method is to overwhelm the endogenous biotin with a high dose of targeting moiety-streptavidin or -avidin conjugate. Another method is a pretreatment with an amount of avidin sufficient to bind substantially all of a recipient's endogenous biotin. In conducting this method, avidin may be administered intravenously, orally or by enema. Alternatively, the recipient may be placed on a biotin-free diet prior to conducting a pretargeting protocol. Another method to address endogenous biotin employs oral, non-absorbable antibiotics.

Cytokines, such as interleukins (e.g., IL-2 and IL-4), colony stimulating factors (e.g., GM-CSF), interferons, (e.g., interferon-gamma), and tumor necrosis factor (TNF), may be employed as anti-tumor active agents in the practice of pretargeting protocols of the present invention. In addition, the pretargeting protocols of the present invention have applications with respect to additional conditions. Immunosuppressive cytokines, such as TGF-beta, may be employed, for example, in the treatment of autoimmune diseases; such as rheumatoid arthritis, systemic lupus erythematosus, insulin-dependent diabetes mellitus, multiple sclerosis, pulmonary fibrosis and the like; tissue transplantation facilitation in liver and kidney tissues, for example; obviation or prevention of graft-versus-host reaction; and the like.

### Brief Description of the Drawing

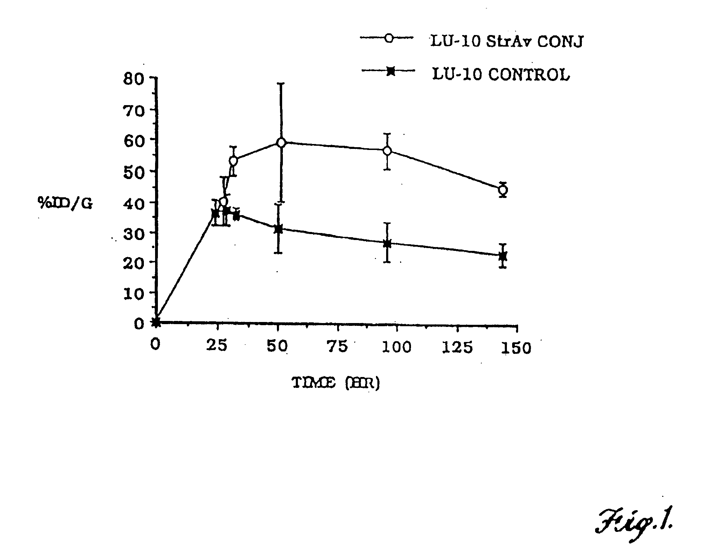

Figure 1 depicts the tumor uptake profile of NR-LU-10-streptavidin conjugate (LU-10-StrAv) in comparison to a control profile of native NR-LU-10 whole antibody.

### Detailed Description of the Invention

Prior to setting forth the invention, it may be helpful to set forth definitions of certain terms to be used within the disclosure.

Targeting moiety: A molecule that binds to a defined population of cells. The targeting moiety may bind a receptor, an oligonucleotide, an enzymatic substrate, an antigenic determinant, or other binding site present on or in the target cell population. Antibody is used throughout the specification as a prototypical example of a targeting moiety. Antibody fragments and small peptide sequences capable of recognizing expressed antigen are also contemplated targeting moieties within the present invention. Tumor is used as a prototypical example of a target in describing the present invention.

Ligand/anti-ligand pair: A complementary/anti-complementary set of molecules that demonstrate specific binding, generally of relatively high affinity. Exemplary ligand/anti-ligand pairs include zinc finger protein/dsDNA fragment, enzyme/inhibitor, hapten/antibody, lectin/carbohydrate, ligand/receptor, and biotin/avidin. Lower molecular weight forms of the ligand or anti-ligand molecules that bind with complementary anti-ligands or ligands are also contemplated by the present invention. Biotin/avidin is used throughout the specification as a prototypical example of a ligand/anti-ligand pair.

Anti-ligand: As defined herein, an "anti-ligand" demonstrates high affinity, and preferably, multivalent binding of the complementary ligand. Preferably, the anti-ligand is large enough to avoid rapid renal clearance, and contains sufficient multivalency to accomplish crosslinking and aggregation of targeting moiety-ligand conjugates. Univalent anti-ligands are also contemplated by the present invention. Anti-ligands of the present invention may exhibit or be derivatized to exhibit structural features that direct the uptake thereof, e.g., galactose residues that direct liver uptake. Avidin and streptavidin are used herein as prototypical anti-ligands.

Avidin: As defined herein, "avidin" includes avidin, streptavidin and derivatives and analogs thereof that are capable of high aff inity, multivalent or univalent binding of biotin.

Ligand: As defined herein, a "ligand" is a relatively small, soluble molecule that exhibits rapid serum, blood and/or whole body clearance when administered intravenously in an animal or human. Biotin is used as the prototypical ligand.

Active Agent: A diagnostic or therapeutic agent ("the payload"), including radionuclides, drugs, anti-tumor agents, toxins and the like. Radionuclide therapeutic agents are used as prototypical active agents.

NₓS_{y} Chelates: As defined herein, the term "NₓS_{y} chelates" includes buoy chelators that are capable of
(i) coordinately binding a metal or radiometal and
(ii) covalently attaching to a targeting moiety, ligand or anti-ligand. Particularly preferred NₓS_{y} chelates have N₂S₂ and N₃S cores. Exemplary NₓS_{y} chelates are described in Fritzberg et al., Proc. Natl. Acad. Sci. USA 85:4024-29, 1988; in Weber et al., Bioconj. Chem. 1:431-37, 1990; and in the references cited therein, for instance.

Pretargeting: As defined herein, pretargeting involves target site localization of a targeting moiety that is conjugated with one member of a ligand/anti-ligand pair; after a time period sufficient for optimal target-to-non-target accumulation of this targeting moiety conjugate, active agent conjugated to the opposite member of the ligand/anti-ligand pair is administered and is bound (directly or indirectly) to the targeting moiety conjugate at the target site (two-step pretargeting).

Clearing Agent: An agent capable of binding, complexing or otherwise associating with an administered moiety (e.g., targeting moiety-ligand, targeting moiety-anti-ligand or anti-ligand alone) present in the recipient's circulation; thereby facilitating circulating moiety clearance from the recipient's body, removal from blood circulation, or inactivation thereof in circulation. The clearing agent is preferably characterized by physical properties, such as size, charge, configuration or a combination thereof, that limit clearing agent access to the population of target cells recognized by a targeting moiety used in the same treatment protocol as the clearing agent.

Target Cell Retention: The amount of time that a radionuclide or other therapeutic agent remains at the target cell surface or within the target cell. Catabolism of conjugates or molecules containing such therapeutic agents appears to be primarily responsible for the loss of target cell retention.

Conjugate: A conjugate encompasses chemical conjugates (covalently or non-covalently bound), fusion proteins and the like.

A recognized disadvantage associated with *in vivo* administration of targeting moiety-radioisotopic conjugates for imaging or therapy is localization of the attached radioactive agent at both non-target and target sites. Until the administered radiolabeled conjugate clears from the circulation, normal organs and tissues are transitorily exposed to the attached radioactive agent. For instance, radiolabeled whole antibodies that are administered *in vivo* exhibit relatively slow blood clearance; maximum target site localization generally occurs 1-3 days post-administration. Generally, the longer the clearance time of the conjugate from the circulation, the greater the radioexposure of non-target organs.

These characteristics are particularly problematic with human radioimmunotherapy. In human clinical trials, the long circulating half-life of radioisotope bound to whole antibody causes relatively large doses of radiation to be delivered to the whole body. In particular, the bone marrow, which is very radiosensitive, is the dose-limiting organ of non-specific toxicity.

In order to decrease radioisotope exposure of non-target tissue, potential targeting moieties generally have been screened to identify those that display minimal non-target reactivity, while retaining target specificity and reactivity. By reducing non-target exposure (and adverse non-target localization and/or toxicity), increased doses of a radiotherapeutic conjugate may be administered; moreover, decreased non-target accumulation of a radiodiagnostic conjugate leads to improved contrast between background and target.

Therapeutic drugs, administered alone or as targeted conjugates, are accompanied by similar disadvantages. Again, the goal is administration of the highest possible concentration of drug (to maximize exposure of target tissue), while remaining below the threshold of unacceptable normal organ toxicity (due to non-target tissue exposure). Unlike radioisotopes, however, therapeutic drugs need to be taken into a target cell to exert a cytotoxic effect. In the case of targeting moiety-therapeutic drug conjugates, it would be advantageous to combine the relative target specificity of a targeting moiety with a means for enhanced target cell internalization of the targeting moiety-drug conjugate.

In contrast, enhanced target cell internalization is disadvantageous if one administers diagnostic agent-targeting moiety conjugates. Internalization of diagnostic conjugates results in cellular catabolism and degradation of the conjugate. Upon degradation, small adducts of the diagnostic agent or the diagnostic agent per se may be released from the cell, thus eliminating the ability to detect the conjugate in a target-specific manner.

One method for reducing non-target tissue exposure to a diagnostic or therapeutic agent involves site, "pretargeting" the targeting moiety at a target site, and then subsequently administering a rapidly clearing diagnostic or therapeutic agent conjugate that is capable of binding to the "pretargeted" targeting moiety at the target site. A description of some embodiments of the pretargeting technique may be found in US Patent No. 4,863,713 (Goodwin et al.).

The "two-step" pretargeting procedures feature targeting moiety-ligand or targeting moiety-anti-ligand administration, followed by administration of active agent conjugated to the opposite member of the ligand-anti-ligand pair. As an optional step "1.5" in the two-step pretargeting methods of the present invention, a clearing agent (preferably other than ligand or anti-ligand alone) is administered to facilitate the clearance of circulating targeting moiety-containing conjugate.

In the two-step pretargeting approach, the clearing agent preferably does not become bound to the target cell population, either directly or through the previously administered and target cell bound targeting moiety-anti-ligand or targeting moiety-ligand conjugate. An example of two-step pretargeting involves the use of biotinylated human transferrin as a clearing agent for avidin-targeting moiety conjugate, wherein the size of the clearing agent results in liver clearance of transferrin-biotin-circulating avidin-targeting moiety complexes and substantially precludes association with the avidin-targeting moiety conjugates bound at target cell sites. (See, Goodwin, D.A., Antibod. Immunoconj. Radiopharm., 4: 427-34, 1991).

In one aspect of the present invention, a targeting moiety-anti-ligand conjugate is administered *in vivo*; upon target localization of the targeting moiety-anti-ligand conjugate (i.e., and clearance of this conjugate from the circulation), an active agent-ligand conjugate is parenterally administered. This method enhances retention of the targeting moiety-anti-ligand : ligand-active agent complex at the target cell (as compared with targeting moiety-ligand : anti-ligand : ligand-active agent complexes and targeting moiety-ligand : anti-ligand-active agent complexes). Although a variety of ligand/anti-ligand pairs may be suitable for use within the claimed invention, a preferred ligand/anti-ligand pair is biotin/avidin.

In a second aspect of the invention, radioiodinated biotin and related methods are disclosed. Previously, radioiodinated biotin derivatives were of high molecular weight and were difficult to characterize. The radioiodinated biotin described herein is a low molecular weight compound that has been easily and well characterized.

In a third aspect of the invention, a targeting moiety-ligand conjugate is administered *in vivo*; upon target localization of the targeting moiety-ligand conjugate (i.e., and clearance of this conjugate from the circulation), a drug-anti-ligand conjugate is parenterally administered. This two-step method not only provides pretargeting of the targeting moiety conjugate, but also induces internalization of the subsequent targeting moiety-ligand-anti-ligand-drug complex within the target cell.

The "targeting moiety" of the present invention binds to a defined target cell population, such as tumor cells. Preferred targeting moieties useful in this regard include antibody and antibody fragments, peptides, and hormones. Proteins corresponding to known cell surface receptors (including low density lipoproteins, transferrin and insulin), fibrinolytic enzymes, anti-HER2, platelet binding proteins such as annexins, and biological response modifiers (including interleukin, interferon, erythropoietin and colony-stimulating factor) are also preferred targeting moieties. Also, anti-EGF receptor antibodies, which internalize following binding to the receptor and traffic to the nucleus to an extent, are preferred targeting moieties for use in the present invention to facilitate delivery of Auger emitters and nucleus binding drugs to target cell nuclei.
Oligonucleotides, e.g., antisense oligonucleotides that are complementary to portions of target cell nucleic acids (DNA or RNA), are also useful as targeting moieties in the practice of the present invention. Oligonucleotides binding to cell surfaces are also useful. Analogs of the above-listed targeting moieties that retain the capacity to bind to a defined target cell population may also be used within the claimed invention. In addition, synthetic targeting moieties may be designed.

Functional equivalents of the aforementioned molecules are also useful as targeting moieties of the present invention. One targeting moiety functional equivalent is a "mimetic" compound, an organic chemical construct designed to mimic the proper configuration and/or orientation for targeting moiety-target cell binding. Another targeting moiety functional equivalent is a short polypeptide designated as a "minimal" polypeptide, constructed using computer-assisted molecular modeling and mutants having altered binding affinity, which minimal polypeptides exhibit the binding affinity of the targeting moiety.

Preferred targeting moieties of the present invention are antibodies (polyclonal or monoclonal), peptides, oligonucleotides or the like. Polyclonal antibodies useful in the practice of the present invention are polyclonal (Vial and Callahan, Univ. Mich. Med. Bull., 20: 284-6, 1956), affinity-purified polyclonal or fragments thereof (Chao et al., Res. Comm. in Chem. Path. & Pharm., 9: 749-61, 1974).

Monoclonal antibodies useful in the practice of the present invention include whole antibody and fragments thereof. Such monoclonal antibodies and fragments are producible in accordance with conventional techniques, such as hybridoma synthesis, recombinant DNA techniques and protein synthesis. Useful monoclonal antibodies and fragments may be derived from any species (including humans) or may be formed as chimeric proteins which employ sequences from more than one species. See, generally, Kohler and Milstein, Nature, 256: 495-97, 1975; Eur. J. Immunol., 6: 511-19, 1976.

Human monoclonal antibodies or "humanized" murine antibody are also useful as targeting moieties in accordance with the present invention. For example, murine monoclonal antibody may be "humanized" by genetically recombining the nucleotide sequence encoding the murine Fv region (i.e., containing the antigen binding sites) or the complementarity determining regions thereof with the nuclectide sequence encoding a human constant domain region and an Fc region, e.g., in a manner similar to that disclosed in European Patent Application No. 0,411,893 A2. Some murine residues may also be retained within the human variable region framework domains to ensure proper target site binding characteristics. Humanized targeting moieties are recognized to decrease the immunoreactivity of the antibody or polypeptide in the host recipient, permitting an increase in the half-life and a reduction in the possibility of adverse immune reactions.

Types of active agents (diagnostic or therapeutic) useful herein include, anti-tumor agents.

Other anti-tumor agents,e.g., agents active against proliferating cells, are administrable in accordance with the present invention. Exemplary anti-tumor agents include cytokines and other moieties, such as interleukins (e.g.,IL-2, IL-4, IL-6, IL-12 and the like), transforming growth factor-beta, lymphotoxin, tumor necrosis factor, interferons (e.g., gamma-interferon), colony stimulating factors (e.g., GM-CSF, M-CSF and the like), vascular permeability factor or the like, lectin inflammatory response promoters (selectins), such as L-selectin, E-selectin, P-selectin or the like, proteinaceous moieties such as Clq and NK receptor protein, and like molecules.

Ligands suitable for use within the present invention include biotin, haptens, lectins, epitopes, dsDNA fragments, enzyme inhibitors and analogs and derivatives thereof. Useful complementary anti-ligands include avidin (for biotin), carbohydrates (for lectins) and antibody, fragments or analogs thereof, including mimetics (for haptens and epitopes) and zinc finger proteins (for dsDNA fragments) and enzymes (for enzyme inhibitors). Preferred ligands and anti-ligands bind to each other with an affinity of at least about k_{D} ≥ 10⁹ M.

One component to be administered in a preferred two-step pretargeting protocol is a targeting moiety-anti-ligand or a targeting moiety-ligand conjugate.

A preferred targeting moiety useful in these embodiments of the present invention is a monoclonal antibody. Protein-protein conjugations are generally problematic due to the formation of undesirable byproducts, including high molecular weight and cross-linked species, however. A non-covalent synthesis technique involving reaction of biotinylated antibody with streptavidin has been reported to result in substantial byproduct formation. Also, at least one of the four biotin binding sites on the streptavidin is used to link the antibody and streptavidin, while another such binding site may be sterically unavailable for biotin binding due to the configuration of the streptavidin-antibody conjugate.

Thus, covalent streptavidin-antibody conjugation is preferred, but high molecular weight byproducts are often obtained. The degree of crosslinking and aggregate formation is dependent upon several factors, including the level of protein derivitization using heterobifunctional crosslinking reagents. Sheldon et al., Appl. Radiat. Isot. 43: 1399-1402, 1992, discuss preparation of covalent thioether conjugates by reacting succinimidyl 4-(N-maleimidomethyl)cyclohexane -1-carboxylate (SMCC)-derivatized antibody and iminothiolane-derivatized streptavidin.

Streptavidin-proteinaceous targeting moiety conjugates are preferably prepared as described in Example I below, with the preparation involving the steps of: preparation of SMCC-derivatized streptavidin; preparation of DTT-reduced proteinaceous targeting moiety; conjugation of the two prepared moieties; and purification of the monosubstituted or disubstituted (with respect to streptavidin) conjugate from crosslinked (antibody-streptavidin-antibody) and aggregate species and unreacted starting materials. The purified fraction is preferably further characterized by one or more of the following techniques: HPLC size exclusion, SDS-PAGE, immunoreactivity, biotin binding capacity and in vivo studies.

Alternatively, thioether conjugates useful in the practice of the present invention may be formed using other thiolating agents, such as SPDP, iminothiolane, SATA or the like, or other thio-reactive heterobifunctional cross linkers, such as m-maleimidobenzoyl-N-hydroxysuccinimide ester, N-succinimidyl(4-iodoacetyl)aminobenzoate or the like.

Streptavidin-proteinaceous targeting moiety conjugates of the present invention can also be formed by conjugation of a lysine epsilon amino group of one protein with a maleimide-derivatized form of the other protein. For example, at pH 8-10, lysine epsilon amino moieties react with protein maleimides, prepared, for instance, by treatment of the protein with SMCC, to generate stable amine covalent conjugates. In addition, conjugates can be prepared by reaction of lysine epsilon amino moieties of one protein with aldehyde functionalities of the other protein. The resultant imine bond is reducible to generate the corresponding stable amine bond. Aldehyde functionalities may be generated, for example, by oxidation of protein sugar residues or by reaction with aldehyde-containing heterobifunctional cross linkers.

Another method of forming streptavidin-targeting moiety conjugates involves immobilized iminobiotin that binds SMCC-derivatized streptavidin. In this conjugation/purification method, the reversible binding character of iminobiotin (immobilized) to streptavidin is exploited to readily separate conjugate from the unreacted targeting moiety. Iminobiotin binding can be reversed under conditions of lower pH and elevated ionic strength, e.g., NH₂OAc, pH 4 (50 mM) with 0.5 M NaCl.

For streptavidin, for example, the conjugation/purification proceeds as follows:
- SMCC-derivatized streptavidin is bound to immobilized iminobiotin (Pierce Chemical Co., St. Louis, Missouri), preferably in column format;
- a molar excess (with respect to streptavidin) of DTT-reduced antibody (preferably free of reductant) is added to the nitrogen-purged, phosphate-buffered iminobiotin column wherein the SMCC-streptavidin is bound (DTT-reduced antibody will saturate the bound SMCC-streptavidin, and unbound reduced antibody passing through the column can be reused);
- the column is washed free of excess antibody; and
- a buffer that lowers the pH and increases ionic strength is added to the column to elute streptavidin-antibody conjugate in pure form.

As indicated above, targeting moiety-mediated ligand-anti-ligand pretargeting involves the localization of either targeting moiety-ligand or targeting moiety-anti-ligand at target tissue. Often, peak uptake to such target tissue is achieved before the circulating level of targeting moiety-containing conjugate in the blood is sufficiently low to permit the attainment of an optimal target-to-non-target conjugate ratio. To obviate this problem, two approaches are useful. The first approach allows the targeting moiety-containing conjugate to clear from the blood by "natural" or endogenous clearance mechanisms. This method is complicated by variations in systemic clearance of proteins and by endogenous ligand or anti-ligand. For example, endogenous biotin may interfere with the preservation of biotin binding sites on a streptavidin-targeting moiety conjugate.

The second approach for improving targeting moiety-ligand or targeting moiety-anti-ligand conjugate target-to-blood ratio "chases" the conjugate from the circulation through in vivo complexation of conjugate with a molecule constituting or containing the complementary anti-ligand or ligand. When biotinylated antibodies are used as a ligand-targeting moiety conjugate, for example, avidin forms relatively large aggregated species upon complexation with the circulating biotinylated antibody, which aggregated species are rapidly cleared from the blood by the RES uptake. See, for example, U.S. Patent No. 4,863,713. One problem with this method, however, is the potential for cross-linking and internalizing tumor-bound biotinylated antibody by avidin.

When avidin-targeting moiety conjugates are employed, poly-biotinylated transferrin has been used to form relatively large aggregated species that are cleared by RES uptake. See, for example, Goodwin, J. Nucl. Med. 33(10):1816-18, 1992). Poly-biotinylated transferrin also has the potential for cross-linking and internalizing tumor-bound avidinylated-targeting moiety, however. In addition, both "chase" methodologies involve the prolonged presence of aggregated moieties of intermediate, rather than large, size (which are not cleared as quickly as large size particles by RES uptake), thereby resulting in serum retention of subsequently administered ligand-active agent or anti-ligand-active agent. Such serum retention unfavorably impacts the target cell-to-blood targeting ratio.

The present invention provides clearing agents of protein and non-protein composition having physical properties facilitating use for *in vivo* complexation and blood clearance of anti-ligand/ligand (e.g., avidin/biotin)-targeting moiety (e.g., antibody) conjugates. These clearing agents are useful in improving the target:blood ratio of targeting moiety conjugate. Other applications of these clearing agents include lesional imaging or therapy involving blood clots and the like, employing antibody-active agent delivery modalities. For example; efficacious anti-clotting agent provides rapid target localization and high target:non-target targeting ratio. Active agents administered in pretargeting protocols of the present invention using efficient clearing agents are targeted in the desirable manner and are, therefore, useful in the imaging/therapy of conditions such as pulmonary embolism and deep vein thrombosis.

Clearing agents useful in the practice of the present invention preferably exhibit one or more of the following characteristics:
- rapid, efficient complexation with targeting moiety-ligand (or anti-ligand) conjugate *in vivo*;
- rapid clearance from the blood of targeting moiety conjugate capable of binding a subsequently administered complementary anti-ligand or ligand containing molecule;
- high capacity for clearing (or inactivating) large amounts of targeting moiety conjugate; and
- low immunogenicity.

Preferred clearing agents include hexose-based and non-hexose based moieties. Hexose-based clearing agents are molecules that have been derivatized to incorporate one or more hexoses (six carbon sugar moieties) recognized by Ashwell receptors or other receptors such as the mannose/N-acetylglucosamine receptor which are associated with endothelial cells and/or Kupffer cells of the liver or the mannose 6-phosphate receptor. Exemplary of such hexoses are galactose, mannose, mannose 6-phosphate, N-acetylglucosamine and the like. Other moieties recognized by Ashwell receptors, including glucose, N-galactosamine, N-acetylgalactosamine, thioglycosides of galactose and, generally, D-galactosides and glucosides or the like may also be used in the practice of the present invention. Galactose is the prototypical clearing agent hexose derivative for the purposes of this description. Galactose thioglycoside conjugation to a protein is preferably accomplished in accordance with the teachings of Lee et al., "2-Imino-2-methoxyethyl 1-Thioglycosides: New Reagents for Attaching Sugars to Proteins," Biochemistry, 15(18): 3956, 1976. Another useful galactose thioglycoside conjugation method is set forth in Drantz et al, "Attachment of Thioglycosides to Proteins: Enhancement of Liver Membrane Binding," Biochemistry, 15(18): 3963, 1976. Thus, galactose-based and non-galactose based molecules are discussed below.

Protein-type galactose-based clearing agents include proteins having endogenous exposed galactose residues or which have been derivatized to expose or incorporate such galactose residues. Exposed galactose residues direct the clearing agent to rapid clearance by endocytosis into the liver through specific receptors therefor (Ashwell receptors). These receptors bind the clearing agent, and induce endocytosis into the hepatocyte, leading to fusipn with a lysosome and recycle of the receptor back to the cell surface. This clearance mechanism is characterized by high efficiency, high capacity and rapid kinetics.

An exemplary clearing agent of the protein-based/galactose-bearing variety is the asialoorosomucoid derivative of human alpha-1 acid glycoprotein (orosomucoid, molecular weight = 41,000 Dal, isoelectric point = 1.8-2.7). The rapid clearance from the blood of asialoorosomucoid has been documented by Galli, et al., J. of Nucl. Med. Allied Sci. 32(2): 110-16, 1988.

Treatment of orosomucoid with neuraminidase removes sialic acid residues, thereby exposing galactose residues. Other such derivatized clearing agents include, for example, galactosylated albumin, galactosylated-IgM, galactosylated-IgG, asialohaptoglobin, asialofetuin, asialoceruloplasmin and the like.

Human serum albumin (HSA), for example, may be employed in a clearing agent of the present invention as follows:
(Hexose)ₘ--Human Serum Albumin (HSA)--(Ligand)ₙ , wherein n is an integer from 1 to about 10 and m is an integer from 1 to about 25 and wherein the hexose is recognized by Ashwell receptors.
In a preferred embodiment of the present invention the ligand is biotin and the hexose is galactose. More preferably, HSA is derivatized with from 10-20 galactose residues and 1-5 biotin residues. Still more preferably, HSA clearing agents of the present invention are derivatized with from about 12 to about 15 galactoses and 3 biotins. Derivatization with both galactose and biotin are conducted in a manner sufficient to produce individual clearing agent molecules with a range of biotinylation levels that averages a recited whole number, such as 1, biotin. Derivatization with 3 biotins, for example, produces a product mixture made up of individual clearing agent molecules, substantially all of which having at least one biotin residue. Derivatization with 1 biotin produces a clearing agent product mixture, wherein a significant portion of the individual molecules are not biotin derivatized. The whole numbers used in this description refer to the average biotinylation of the clearing agents under discussion.

In addition, clearing agents based upon human proteins, especially human serum proteins, such as, for example, orosomucoid and human serum albumin, human IgG, human-anti-antibodies of IgG and IgM class and the like, are less immunogenic upon administration into the serum of a human recipient. Another advantage of using asialoorosomucoid is that human orosomucoid is commercially available from, for example, Sigma Chemical Co, St. Louis, Missouri.

One way to prevent clearing agent compromise of target-bound conjugate through direct complexation is through use of a clearing agent of a size sufficient to render the clearing agent less capable of diffusion into the extravascular space and binding to target-associated conjugate. This strategy is useful alone or in combination with the aforementioned recognition that exposed galactose residues direct rapid liver uptake. This size-exclusion strategy enhances the effectiveness of non-galactose-based clearing agents of the present invention. The combination (exposed galactose and size) strategy improves the effectiveness of "protein-type" or "polymer-type" galactose-based clearing agents.

Galactose-based clearing agents include galactosylated, biotinylated proteins (to remove circulating streptavidin-targeting moiety conjugates, for example) of intermediate molecular weight (ranging from about 40,000 to about 200,000 Dal), such as biotinylated asialoorosomucoid, galactosyl-biotinyl-human serum albumin or other galactosylated and biotinylated derivatives of non-immunogenic soluble natural proteins, as well as biotin- and galactose-derivatized polyglutamate, polylysine, polyarginine, polyaspartate and the like. High molecular weight moieties (ranging from about 200,000 to about 1,000,000 Dal) characterized by poor target access, including galactosyl-biotinyl-IgM or -IgG (approximately 150,000 Dal) molecules, as well as galactose- and biotin-derivatized transferrin conjugates of human serum albumin, IgG and IgM molecules and the like, can also be used as clearing agents of the claimed invention. Chemically modified polymers of intermediate or high molecular weight (ranging from about 40,000 to about 1,000,000 Dal), such as galactose- and biotin-derivatized dextran, hydroxypropylmethacrylamide polymers, polyvinylpyrrolidone-polystyrene copolymers, divinyl ether-maleic acid copolymers, pyran copolymers, or PEG, also have utility as clearing agents in the practice of the present invention. In addition, rapidly clearing biotinylated liposomes (high molecular weight moieties with poor target access) can be derivatized with galactose and biotin to produce clearing agents for use in the practice of the present invention.

A further class of clearing agents useful in the present invention involve small molecules (ranging from about 500 to about 10,000 Dal) derivatized with galactose and biotin that are sufficiently polar to be confined to the vascular space as an in vivo volume of distribution. More specifically, these agents exhibit a highly charged structure and, as a result, are not readily distributed into the extravascular volume, because they do not readily diffuse across the lipid membranes lining the vasculature. Exemplary of such clearing agents are mono- or poly-biotin-derivatized 6,6'-[(3,3'-dimethyl[1,1'-biphenyl]-4,4'-diyl)bis(azo) bis[4-amino-5-hydroxy-1,3-naphthalene disulfonic acid] tetrasodium salt, mono- or poly-biotinyl-galactose-derivatized polysulfated dextran-biotin, mono- or poly-biotinyl-galactose-derivatized dextran-biotin and the like.

The galactose-exposed or -derivatized clearing agents are preferably capable of (1) rapidly and efficiently complexing with the relevant ligand- or anti-ligand-containing conjugates via ligand-anti-ligand affinity; and (2) clearing such complexes from the blood via the galactose receptor, a liver specific degradation system, as opposed to aggregating into complexes that are taken up by the generalized RES system, including the lung and spleen. Additionally, the rapid kinetics of galactose-mediated liver uptake, coupled with the affinity of the ligand-anti-ligand interaction, allow the use of intermediate or even low molecular weight carriers.

Non-galactose residue-bearing moieties of low or intermediate molecular weight (ranging from about 40,000 to about 200,000 Dal) localized in the blood may equilibrate with the extravascular space and, therefore, bind directly to target-associated conjugate, compromising target localization. In addition, aggregation-mediated clearance mechanisms operating through the RES system are accomplished using a large stoichiometric excess of clearing agent. In contrast, the rapid blood clearance of galactose-based clearing agents used in the present invention prevents equilibration, and the high affinity ligand-anti-ligand binding allows the use of low stoichiometric amounts of such galactose-based clearing agents. This feature further diminishes the potential for galactose-based clearing agents to compromise target-associated conjugate, because the absolute amount of such clearing agent administered is decreased.

Galactose- and biotin-derivatized clearing agents were examined specifically (1) asialoorosomucoid-biotin, (2) human serum albumin derivatized with galactose and biotin, and (3) a 70,000 dalton molecular weight dextran derivatized with both biotin and galactose. The experimentation showed that proteins and polymers are derivatizable to contain both galactose and biotin and that the resultant derivatized molecule is effective in removing circulating streptavidin-protein conjugate from the serum of the recipient. Biotin loading was varied to determine the effects on both clearing the blood pool of circulating avidin-containing conjugate and the ability to deliver a subsequently administered by biotinylated isotope to a target site recognized by the streptavidin-containing conjugate. The effect of relative doses of the administered components with respect to clearing agent efficacy was also examined.

Protein-type and polymer-type non-galactose-based clearing agents include the agents described above, absent galactose exposure or derivitization and the like. These clearing agents act through an aggregation-mediated RES mechanism. In these embodiments of the present invention, the clearing agent used will be selected on the basis of the target organ to which access of the clearing agent is to be excluded. For example, high molecular weight (ranging from about 200,000 to about 1,000,000 Dal) clearing agents will be used when tumor targets or clot targets are involved.

Another class of clearing agents includes agents that do not remove circulating ligand or anti-ligand/targeting moiety conjugates, but instead "inactivate" the circulating conjugates by blocking the relevant anti-ligand or ligand binding sites thereon. These "cap-type" clearing agents are preferably small (500 to 10,000 Dal) highly charged molecules, which exhibit physical characteristics that dictate a volume of distribution equal to that of the plasma compartment (i.e., do not extravasate into the extravascular fluid volume). Exemplary cap-type clearing agents are poly-biotin-derivatized 6,6'-[(3,3'-dimethyl[1,1'-biphenyl]-4,4'-diyl)bis(azo) bis[4-amino-5-hydroxy-1,3-naphthalene disulfonic acid] tetrasodium salt, poly-biotinyl-derivatized polysulfated dextran-biotin, mono- or poly-biotinyl-derivatized dextran-biotin and the like.

Cap-type clearing agents are derivatized with the relevant anti-ligand or ligand, and then administered to a recipient of previously administered ligand/ or anti-ligand/targeting moiety conjugate. Clearing agent-conjugate binding therefore diminishes the ability of circulating conjugate to bind any subsequently administered active agent-ligand or active agent-anti-ligand conjugate. The ablation of active agent binding capacity of the circulating conjugate increases the efficiency of active agent delivery to the target, and increases the ratio of target-bound active agent to circulating active agent by preventing the coupling of long-circulating serum protein kinetics with the active agent. Also, confinement of the clearing agent to the plasma compartment prevents compromise of target-associated ligand or anti-ligand.

Clearing agents of the present invention may be administered in single or multiple doses. A single dose of biotinylated clearing agent, for example, produces a rapid decrease in the level of circulating targeting moiety-streptavidin, followed by a small increase in that level, presumably caused, at least in part, by re-equilibration of targeting moiety-streptavidin within the recipient's physiological compartments. A second or additional clearing agent doses may then be employed to provide supplemental clearance of targeting moiety-streptavidin. Alternatively, clearing agent may be infused intravenously for a time period sufficient to clear targeting moiety-streptavidin in a continuous manner.

Other types of clearing agents and clearance systems are also useful in the practice of the present invention to remove circulating targeting moiety-ligand or -anti-ligand conjugate from the recipient's circulation.

U.S. Patent No. 4,867,962 issued to Abrams describes an improved method for delivering active agent to target sites, which method employs active agent-targeting moiety conjugates. Briefly, the Abrams method contemplates administration to a recipient of two or more active agent-targeting moiety conjugates, wherein each conjugate includes a targeting moiety of a different antibody species. Each of the utilized antibody species is reactive with a different target site epitope (associated with the same or a different target site antigen) , and the patterns of cross-reactivity for the antibody species are non-overlapping. The active agent component of each administered conjugate may be the same or different.

In this manner, the different antibodies (along with the agents attached thereto) accumulate additively at the desired target site, while only one or fewer than the total administered antibody species accumulate on each type of cross-reactive non-target tissue. A higher percentage of the administered agent therefore becomes localized *in vivo* at target sites compared to non-target tissues. For diagnostic agents, this methodology results in more clearly detected or imaged target sites against a comparatively lower (i.e., more diffuse background of non-target tissue accumulation. Lower accumulation of therapeutic agents at different non-target tissues permits larger doses of the associated active agents to be administered without incidence of undesirable non-target toxicity.

The present invention provides two-step pretargeting methods as set forth below.

The two-step approach involves:
administering to the recipient a first conjugate comprising a targeting moiety'of a first antibody species having a first pattern of cross-reactivity and a member of a ligand-anti-ligand binding pair;
administering to the recipient one or more additional targeting conjugates, each such conjugate comprising a targeting moiety of a different antibody species from the species of the first conjugate and from each other and having a substantially non-overlapping pattern of cross-reactivity from each other and from the first pattern of cross-reactivity and the member of the ligand-anti-ligand pair bound to the first conjugate;
optionally administering to the recipient a clearing agent capable of directing the clearance of circulating conjugate from the recipient or optionally treating the recipient with a clearing device or an alternative clearing procedure to substantially remove circulating conjugate from the recipient; and
administering to the recipient a second conjugate comprising an active agent and a ligand/anti-ligand binding pair member, wherein the second conjugate binding pair member is complementary to that of the first conjugate.

One alternative to the optional clearance step set forth above is simply to allow an amount of time to pass that is sufficient to permit the recipient's native clearance mechanisms to substantially remove circulating conjugate.

Alternatively, antibody-based or non-antibody-based targeting moieties may be employed to deliver a ligand or an anti-ligand to a target site bearing an unregulated antigen. Preferably, a natural binding agent for such an unregulated antigen is used for this purpose. For example, diseases such as hepatoma or myeloma are generally characterized by unregulated IL-6 receptors for which IL-6 acts as an autocrine or paracrine moiety with respect to rapid proliferation of these target cell types. For the treatment of such ailments, IL-6 may therefore be employed as a targeting moiety in a pretargeting protocol of the present invention.

For example, IL-6 and streptavidin may be conjugated via chemical means or be formed as a recombinant molecule. The IL-6-streptavidin conjugate is administered to a recipient, and the IL-6 component of the conjugate directs the localization of the conjugate to IL-6 receptors. This localization will occur preferentially to sites bearing unregulated IL-6 receptors. After target site localization occurs, a clearing agent is optionally administered to substantially clear the recipient's circulation of IL-6-streptavidin conjugate. Suitable clearing agents for this purpose are, for example, IL-6 receptor-HSA-galactose; anti-IL-6-antibody-HSA-galactose or the like. After a time sufficient for substantial clearance of IL-6 from the recipient's circulation, active agent-biotin conjugate is administered and localizes to target sites via the IL-6-streptavidin conjugate.

In the practice of these aspects of the present invention, the target site accretion of active agent conjugate receptor (i.e., the ligand or anti-ligand conjugated to the first antibody species and additional targeting antibody species) is improved, because each antibody species recognizes a different epitope associated with the target site. This alternative epitope approach provides more potential target site binding points for the active agent conjugate receptor. Consequently, actual or effective target site saturation (via epitope saturation and/or steric hindrance) may be avoided; the target binding site barrier (binding of surface epitopes of the target site, which limits egress to internal target site epitopes) may be circumvented; and additive accumulation of active agent conjugate receptor may be accomplished. The practice of this aspect of the present invention provides a ligand- or anti-ligand-active agent conjugate with a target site that is densely populated with the anti-ligand or ligand antigen to which the active agent-containing conjugate may bind. Up to a point dictated primarily by steric factors, increased target site antigen density facilitates increased active agent accretion thereto.

The phrase "non-overlapping patterns of cross-reactivity" indicates that the non-target tissues bound by one antibody species differs substantially from the non-target tissues bound by another antibody species. The patterns of cross-reactivity must differ to the extent necessary to proportionately reduce the background for diagnostic applications and to reduce the toxicity to normal tissues for therapeutic applications. The less overlap in cross-reactivity to non-target tissues, the more useful an antibody pair (or larger set of antibodies) in the practice of these aspects of the present invention.

The patterns of cross-reactivity for monoclonal antibodies directed against a particular target site are analyzed to identify a set of two or more target-specific monoclonal antibodies with non-overlapping cross-reactivity for use in a diagnostic or therapeutic application. Antibodies may be screened by a variety of methods. The *in vitro* procedure employed to determine reactivity with target tissue and cross-reactivity with non-target tissue is immunohistochemical analysis. Tissues to which the antibody species binds are identified by exposing the tissue to the antibody; washing the tissue to remove any unbound antibody; and detecting the presence of bound antibody. Frozen tissue sections are preferred for use in these immunohistochemical methods, because tissue fixation may destroy epitopes and is associated with uncertainties in timing that may compromise antigen preservation. For antigens that are known to be preserved following fixation, such a technique may be effectively used. *in vitro* histochemical procedures are known (e.g., Ceriani et al., Cancer Research, 47: 532-540, 1987 or Example I of U.S. Patent No. 4,867,962).

In these multi-targeting moiety administering aspects of the present invention, the doses of each administered component will be determined by the attending physician in accordance with his or her experience, the particulars of the recipient's condition (the nature and location of the target site, including the antigens associated therewith, will impact antibody species selection and route of administration decisions) and the combination of antibody species to be employed (antibody performance varies with respect to antigen density and the affinity of the antibody for the antigen). It would be evident to one of ordinary skill in the art how to determine useful dosages of the components described above.

It is another object of the invention to provide further improvements of the afore-described pretargeting methods by reducing or eliminating the potential antigenicity of the pretargeting conjugate and/or the active agent containing conjugate.

In particular, this will be accomplished by modification of the ligand or anti-ligand contained in the pretargeting conjugate or active agent containing conjugate, or the targeting moiety contained in the pretargeting conjugate by the attachment of glycol moieties, preferably polyalkylene glycol moieties, and more preferably polyethylene glycol moieties, to one or more of the anti-ligand or targeting moiety.

Methods for the attachment of glycols, and particularly polyethylene glycols to desired compounds, e.g., proteins, are well known in the art. For example, WO 94/05332 describes means for attachment of glycol moieties to macromolecules including proteins and nucleic acids via glycosylation moieties, preferably using alkylene glycols having 1 to 20 carbon atoms. This is accomplished by activating a polyalkylene glycol, reacting the activated polyalkylene glycol with a diamino compound so as to couple the polyalkylene glycol to the diamino compound via one of its amino groups, oxidizing the macromolecule (e.g., a therapeutic protein) to activate at least one glycol group therein, and reacting the polyalkylene glycol coupled diamino compound with the oxidized glycosyl group, to provide a glycolated glycosylated macromolecule containing a glycol bonded to the macromolecule via glycosyl groups. The reference describes that this results in macromolecules have decreased immunogenic response but maintained biological activity. The described macromolecules include polypeptides, nucleic acids and lipids. Moreover, the reference describes that the glycol derivatized macromolecules maintain binding capacity.

U.S. Patent No. 5,284,934 to Allen, Jr. similarly describes the preparation of carbohydrate-binding lectin derivatives for use as immune modulators or immune conjugates by attachment of polyethylene glycol to *Ricinius communis* agglutin I (PEG-RCAI) to a polymer by use of a coupling agent, e.g., 1, 1-carbonyl-diimidazole. The resultant conjugate is disclosed to be substantially non-immunogenic. The patent further discloses other references which describe attachment of macromolecules, e.g., enzymes and hormones to polyethylene glycol, to produce conjugates for therapeutic use which are substantially non-immunogenic. These references are incorporated-by reference in their entirety.

It is known that PEGylation may result in proteins and polypeptides having reduced immunogenicity and altered serum clearance properties. It is further known that PEG modified protectors are resistant to metabolic deactivation. The following references are representative of PEG modification of proteins. Wang et al., Cancer Res., 53, 4588-4597 (1993), who describe PEG attachment to a chimeric toxin; Rosenberg et al., J. Biol. Chem., 267 (32), 2289-2293 (1992), who describe PEG modification of asialofetuin; Somack et al., Free Rad. Res. Comms., 12-13, 553-562 (1991), who describe PEG modification of superoxide dismutase; Malik et al., Exp. Hematol., 20, 1028-1035 (1992) who describe PEG modification of macrophage colony stimulating factor (GM-CSF) to produce derivatives having conserved biological activity; and Tsutsumi et al., Japan J. Cancer Res., 85, 9-12 (1994) who describe PEG modification of tumor necrosis factor to produce conjugates having improved anti-tumor activity. In some instances PEG modification of a protein has been disclosed to result in loss of biological activity. (*See*, e.g., Wang et al., Id.; Sumack et al, Id. and Tsutsumi et al., Id., in this regard). However, in most instances this can be obviated or alleviated by optimizing the amount of bound glycol residues, the particular means for their attachment, or by the use of protecting agents which protect active sites, e.g., binding sites, during PEGylation. Essentially, in the present invention, the particular ligand, anti-ligand, targeting moiety or active agent will be derivatized with one or more glycol residues, e.g., polyethylene glycol, and then assayed for activity. In the case of the ligand or anti-ligand or targeting moiety this will be determined in binding assays which assay the ability of the glycol derivatized moiety to bind the corresponding anti-ligand or ligand.

In the prototypical embodiment, the moiety to be derivatized with glycol residues will comprise streptavidin or avidin. However, glycol attachment, and more specifically polyethylene glycol attachment, should reduce the immunogenicity of other ligands and anti-ligands, as well as active agents and targeting moieties which are used n pretargeting protocols.

The subject embodiment will comprise particular benefit if the ligand, anti-ligand or active agent is of non-mammalian origin and is therefore likely to induce an immunogenic response. However, PEGylation may also be used to reduce or eliminate the potential immunogenicity of mammalian proteins or to alter their clearance properties. For example, the present invention embraces the attachment of polyethylene glycol moieties to the targeting moiety, which is typically of mammalian origin, e.g., an antibody, antibody fragment or derivative, e.g., single chain antibody, Fab, (Fab)₂, Fv, chimeric antibody, humanized antibody, bispecific antibody, which targeting moiety is contained in the pretargeted conjugate. Such derivatization should eliminate or alleviate the possibility of an adverse immunogenic response, i.e., HAMA response, to the targeting moiety contained in the pretargeted conjugate. While this is not a significant concern in diagnostic applications, such an antibody response may potentially be problematic during therapeutic pretargeting methods since it may prevent the conjugate from being delivered efficiently to the desired target site.

As noted, there are many known procedures for the covalent attachment of glycol moieties, e.g., pclyalkylene glycol moieties, to desired molecules. Such techniques are described in the previously discussed references pertaining to polyalkylene derivatization of macromolecules, e.g., proteins, which are incorporated by reference herein. Additionally, such techniques are described in Delgado et al., Biotech Appl. Biochem., 252 (11), 3578-3581 (1977). Such procedures typically exploit acylation or alkylation reactions involving amino groups, or alternatively attach such moieties to carboxyl residues or to activated (oxidized) glycosyl groups (see, WO 94/05 332).

The selection of the particular means of attachment will depend on the available functional groups contained on the particular ligand, anti-ligand, targeting moiety or active agent which is to be modified by the addition of glycol residues. If suitable functional groups are not available for attachment, they may be introduced on the particular compound to be modified by reaction with compounds containing the desired functional groups, e.g., amino groups, carboxyl groups or glycosyl groups. Means for introduction of functional groups into macromolecules, e.g., proteins and polypeptides are well within the purview of the ordinarily skilled artisan.

A prototypical example of this aspect of the invention relates to conjugation of polyethylene glycol moieties to either avidin or streptavidin. Streptavidin is a 60 Kd tetrameric protein which binds 4 moles of biotin per mole of protein. Preferably, attachment of polyethylene glycol moieties should not substantially affect the ability of streptavidin or. avidin to bind biotin. This will depend upon factors including the particular means and site of polyethylene glycol attachment, and the number of attached polyethylene glycol residues.

As described in detail in the experimental examples relating to this embodiment of the invention, reaction of polyethylene glycol moieties with streptavidin producted streptavidin conjugates containing polyethylene glycol moieties. However, some of such conjugates exhibited substantially reduced biotin binding ability in an HABA assay which measures the displacement of 2-(4'-hydroxy-azobenzene)benzoic acid (HABA) from streptavidin by biotin. However, such problems were substantially alleviated by effectively blocking the biotin binding sites prior to the attachment of polyethylene glycol residues.

Suitable blocking agents include, in particular, the low affinity biotin analogs identified above, antibodies to biotin binding sites, etc. The only prerequisite is that such moieties be capable of removal without adverse effects to the protein and that such attached moieties not adversely affect PEGlyation.

This will preferably be effected by attachment to the particular protein, e.g., streptavidin or avidin, of moieties which reversibly bind to biotin affinity sites, e.g., low affinity biotin analogs.

When targeting moieties, ligands or anti-ligands active agents are attached to polyethylene glycol residues it may also be necessary to protect the active site, e.g., binding site, prior to attachment of polyethylene glycol residues, or to vary the number of polyethylene glycol residues.

Sustained release dosage forms may also be employed in the process of the present invention to deliver active agent to target cells through the pretargeting approach. In this manner, the therapeutic effect of the photosensitizing agent may be achieved over a period of time. Ligand or anti-ligand derivatized liposomes may be employed for this purpose. Hawrot et al., U.S. Patent No. 4,948,590, for example, discuss streptavidinylated liposomes and the encapsulation of active agents therein.

Alternatively, microparticulate or nanoparticulate polymeric bead dosage forms may be employed, for use as clearing agents. In this case, the active agent will be encapsulated in the particulate dosage forms which have a number of ligand or anti-ligand molecules bound thereon. In this manner, active agent is delivered to a target site via ligand-anti-ligand binding and active agent is release at that site over time to provide a sustained therapeutic benefit.

In general, the procedure for forming particulate dosage forms of the present invention involves dissolving the polymer in a halogenated hydrocarbon solvent, dispersing an active agent solution (preferably aqueous) therein, and adding an additional agent that acts as a solvent for the halogenated hydrocarbon solvent but not for the polymer. The polymer precipitates out from the polymer-halogenated hydrocarbon solution onto droplets of the active agent containing solution and entraps the active agent. Preferably the active agent is substantially uniformly dispersed within the sustained release dosage form of the present invention. Following particulate formation, they are washed and hardened with an organic solvent. Water washing and aqueous non-ionic surfactant washing steps follow, prior to drying at room temperature under vacuum.

For biocompatibility purposes, particulate dosage forms, characterized by an active agent dispersed therein in matrix form, are sterilized prior to packaging, storage or administration. Sterilization may be conducted in any convenient manner therefor. For example, the particulates can be irradiated with gamma radiation, provided that exposure to such radiation does not adversely impact the structure or function of the active agent dispersed in the therapeutic agent-polymer matrix or the ligand or anti-ligand attached thereto. If the active agent, ligand or anti-ligand is so adversely impacted, the particulate dosage forms can be produced under sterile conditions.

Release of the active agent from the particulate dosage forms of the present invention can occur as a result of both diffusion and particulate matrix erosion. Biodegradation rate directly impacts active agent release kinetics. The biodegradation rate is regulable by alteration of the composition or structure of the sustained release dosage form. For example, alteration of the lactide/glycolide ratio in preferred dosage forms of the present invention can be conducted, as described by Tice et al., "Biodegradable Controlled-Release Parenteral Systems," Pharmaceutical Technology, pp. 26-35, 1984; by inclusion of polymer hydrolysis modifying agents, such as citric acid and sodium carbonate, as described by Kent et al., "Microencapsulation of Water Soluble Active Polypeptides," U.S. Patent No. 4,675,189; by altering the loading of active agent in the lactide/glycolide polymer, the degradation rate being inversely proportional to the amount of active agent contained therein, and by judicious selection of an appropriate analog of a common family of active agents that core exhibit different potencies so as to alter said core loadings; and by variation of particulate size, as described by Beck et al., "Poly(DL-Lactide-Co-Glycolide)/Norethisterone Microcapsules: An Injectable Biodegradable Contraceptive," Biol. Reprod., 28:186-195, 1983, or the like. All of the aforementioned methods of regulating biodegradation rate influence the intrinsic viscosity of the polymer containing matrix, thereby altering the hydration rate thereof.

The preferred lactide/glycolide structure is biocompatible with the mammalian physiological environment. Also, these preferred sustained release dosage forms have the advantage that biodegradation thereof forms lactic acid and glycolic acid, both normal metabolic products of mammals.

Functional groups required for ligand or anti-ligand bonding to the particles, are optionally included in the particulate structure, along with the non-degradable or biodegradable polymeric units. Functional groups that are exploitable for this purpose include those that are reactive with ligands or anti-ligands such as carboxyl groups, amine groups, sulfhydryl groups and the like. Preferred functional groups include the terminal carboxyl groups of the preferred (lactide-glycolide) polymer containing matrix or the like.

These sustained release dosage forms are also useful with regard to other active agents, such as toxins, chemotherapeutic agents, cytokines and the like.

Monovalent antibody fragment-anti-ligand or - ligand conjugates may be employed in pretargeting protocols of the present invention. For example, a monovalent antibody fragment-streptavidin conjugate may be used to pretarget streptavidin, preferably in additional embodiments of the two-step aspect of the present invention. Exemplary monovalent antibody fragments useful in these embodiments are Fv, Fab, Fab' and the like. Monovalent antibody fragments, typically exhibiting a molecular weight ranging from about 25 kD (Fv) to about 50 kD (Fab, Fab'), are smaller than whole antibody and, therefore, are generally capable of greater target site penetration. Moreover, monovalent binding can result in less binding carrier restriction at the target surface (occurring during use of bivalent antibodies, which bind strongly and adhere to target cell sites thereby creating a barrier to further egress into sublayers of target tissue), thereby improving the homogeneity of targeting.

In addition, smaller molecules are more rapidly cleared from a recipient, thereby decreasing the immunogenicity of the administered small molecule conjugate. A lower percentage of the administered dose of a monovalent fragment conjugate localizes to target in comparison to a whole antibody conjugate. The decreased immunogenicity may permit a greater initial dose of the monovalent fragment conjugate to be administered, however.

A multivalent, with respect to ligand, moiety is preferably then administered. This moiety also has one or more radionuclide associated therewith. As a result, the multivalent moiety serves as both a clearing agent for circulating anti-ligand-containing conjugate (through cross-linking or aggregation of conjugate) and as a therapeutic agent when associated with target bound conjugate. In contrast to the internalization caused by cross-linking described above, cross-linking at the tumor cell surface stabilizes the monovalent fragment-anti-ligand molecule and, therefore, enhances target retention, under appropriate conditions of antigen density at the target cell. In addition, monovalent antibody fragments generally do not internalize as do bivalent or whole antibodies. The difficulty in internalizing monovalent antibodies permits cross-linking by a monovalent moiety serves to stabilize the bound monovalent antibody through multipoint binding. This two-step protocol of the present invention has greater flexibility with respect to dosing, because the decreased fragment immunogenicity allows more streptavidin-containing conjugate, for example, to be administered, and the simultaneous clearance and therapeutic delivery removes the necessity of a separate controlled clearing step.

A potential difficulty in employing two-step pretargeting protocols involving the biotin-avidin or the biotin-streptavidin ligand-anti-ligand pair is the presence of endogenous biotin. Biotin is also known as vitamin H and is present at an endogenous level in mammalian recipients. Mice, for example, have high levels of endogenous biotin, ranging from about microgram to about nanogram concentrations (10⁻⁶ to 10⁻⁹ M). Larger mammals, such as rabbits and dogs, exhibit endogenous biotin at lower levels than mice, ranging from about low nanogram to about high nanogram concentrations (10⁻⁹ to 10⁻¹¹ M). Humans exhibit even lower endogenous biotin levels, ranging from about low picogram to about high picogram concentrations (10⁻¹² to 10⁻¹⁴ M). Because endogenous biotin level is impacted by factors such as diet and suppression of gut wall bacteria by oral antibotics, variability in endogenous biotin level will be observed within each mammalian species.

While the two-step pretargeting methods of the present invention may be conducted despite endogenous biotin, methods of decreasing endogenous biotin or the impact thereof would be useful. One way to diminish the impact of endogenous biotin is to overwhelm the endogenous biotin with a high dose of targeting moiety- streptavidin or -avidin conjugate. More specifically, conjugate is administered in an amount sufficient to substantially bind both the endogenous biotin and sufficient target site epitopes to achieve a diagnostic or therapeutic benefit for the recipient. In the identification of, an appropriate conjugate dose, the endogenous biotin level for each individual recipient may be determined, and the conjugate dose selected accordingly. Alternatively, an appropriate conjugate dose may be based upon average endogenous biotin values for the recipient species.

Another method involves a pretreatment with an amount of avidin sufficient to bind up substantially all of a recipient's endogenous biotin. In this method, avidin may be administered intravenously as a bolus dose followed by slow infusion (e.g., avidin in saline or in PBS), preferably from about 5 minutes to about 30 minutes prior to targeting moiety-streptavidin administration. Alternatively, avidin may be administered orally (e.g., as raw egg whites), preferably earlier and at higher doses than an intravenous administration. Still another alternative is administering a high dose of avidin by enema (e.g., avidin in saline), preferably earlier and at higher doses than intravenous administration.

An intravenously administered agent becomes bioavailable faster than an agent administered via oral or enema routes, therefore generally rendering intravenous administered agents more toxic than the agents administered by oral/enema routes. Also, absorption, distribution, kinetics and metabolism of oral/enema administrations are generally slower than intravenous agent administrations, thereby generally requiring a higher dose for oral/enema administrations.

Alternatively, the recipient may be placed on a biotin-free diet prior to conducting a two-step pretargeting protocol. For example, a mouse recipient of a pretargeting protocol may be placed on a biotin-free diet from about 2 to about 3 days prior to the start of the pretargeting protocol. A larger volume mammal, such as a rabbit or a dog, may be placed on a biotin-free diet from about 3 to about 7 days before the first conjugate administration of a pretargeting protocol of the present invention. A human recipient may be placed on a biotin-free diet (e.g., avoiding dietary sources of biotin such as eggs, nuts, peanut butter, chocolate, candy, yeast, cereals, organ meats such as kidney and liver, meat, meat products, mushrooms, bananas, grapefruit, watermelon, strawberries, beans and legumes) from about 1 to about 2 weeks prior to commencement of the pretargeting protocol. In this manner, the endogenous biotin level of the recipient may be reduced, thereby diminishing any adverse impacts of endogenous biotin on the pretargeting protocol.

An alternative method to address endogenous biotin is the use of oral, non-absorbable antibiotics. Most human endogenous biotin is produced by gut flora (e.g., bacteria, such as E. coli and the like). Potent antibiotics are known which destroy gut flora. Such antibiotics are orally administered and are not absorbed from the intestinal tract, so that they are non-toxic to the recipient. Other functional characteristics of suitable antibiotics are as follows: antagonism of growth and/or survival of one or more species of microorganisms that produce biotin; effectiveness at low doses;and the like. Exemplary of such antibiotics are ampicillin, chloramphenicol, erythromycin, oxacillin, nafcillin, oxytetracycline, penicillin-G, penicillin-V, tetracycline, kanamycin, lincomycin, griseofulvin, doxycycline, novobiocin, colistin, chlortetracycline, and the like. To temporarily lower the level of biotin produced by gut flora, oral, non-absorbable antibiotics, such as may gentamicin, polymyxin-B, vancomycin and the like, may be administered from about 7 to about 10 days prior to the commencement of the pretargeting protocol.

Combinations of the aforementioned methods may be employed in the practice of the present invention. Of the above methods, all may be employed in combination with two-step pretargeting.

It is also expected that administration of the therapeutic conjugate via infusion may be advantageous in therapeutic pretargeting protocols. For example, it should enable administration of a constant low dosage of the active agent, thereby providing for the desired delivery of active agent to target site, but reducing the potential for non-specific binding because of lower average concentration of the particular active agent conjugate which is contained in the patient's circulation. In general, efficacious infusion rates will range from about 10 µg/hr. to about 100 µg/hr.

However, this will of course depend upon the particular active agent, the disease being treated, the condition of patient, the therapeutic regimen (e.g., if other therapies are being utilized in combination with pretargeting protocol), etc. Determination of optimal rates of infusion may be determined by one skilled in the art. It is especially believed that the above divided dosages and constant infusion will be advantageous for therapeutic pretargeting methods involving the administration of radioactive materials and other cytotoxins, e.g., the Y-90-DOTA-biotin conjugate.

Intraarterial administration pretargeting can be applied to targets present in organs or tissues for which supply arteries are accessible. Exemplary applications for intraarterial delivery aspects of the pretargeting methods of the present invention include treatment of liver tumors through hepatic artery administration, brain primary tumors and metastases through carotid artery administration, lung carcinomas through bronchial artery administration and kidney carcinomas through renal artery administration. Intraarterial administration pretargeting can be conducted using chemotherapeutic drug, toxin and anti-tumor active agents as discussed below. High potency drugs, lymphokines, such as IL-2 and tumor necrosis. factor, gamma-interferon, drug/lymphokine-carrier-biotin molecules, biotinylated drugs/lymphokines, and drug/lymphokine/ toxin-loaded, biotin-derivatized liposomes are exemplary of active agents and/or dosage forms useful for the delivery thereof in the practice of this embodiment of the present invention.

The pretargeting protocols set forth above are amenable to use for delivery of other moieties, including anti-tumor agents; chemotherapeutic drugs and the like. For example, most naturally occurring and recombinant cytokines have short *in vivo* half lives. This characteristic limits the clinical effectiveness of these molecules, because near toxic doses are often required. Dose-limiting toxicities in humans have been observed upon high dose IL-2 or tumor necrosis factor, gamma-interferon or lymphotoxin administrations, for example.

Anti-tumor agents, such as IL-2 and TNF, may be employed as active agents in the practice of two-step pretargeting protocols of the present invention. Some anti-tumor agents exhibit short circulation half-lives (less than about 1 hour post-administration), such as IL-2 (half-life of about 10 minutes), other interleukins, TNF, interferons and the like. Such short half-life active agents are amenable to use in pretargeting protocols of the present invention.

Anti-tumor agents having longer half-lives (ranging from about 2 hours to about 12 hours post-administration) are preferably employed at low doses, while conjugates incorporating such moieties at high doses preferably also incorporate a biodistribution directing moiety, such as a polymer, to direct the biodistribution of the conjugate and active agent-containing metabolites thereof to renal excretion. Ligand or anti-ligand derivatization of a long half-life anti-tumor agent may decrease the serum half-life sufficiently to permit higher doses of conjugate to be administered, however.

IL-2 has a molecular weight of 15,500 daltons and is formed of 133 amino acids (42% nonpolar and 58% polar). IL-2 is characterized by 12 free primary amines for reaction with a ligand or an anti-ligand (e.g., biotin or streptavidin) or with functional groups of a biodistribution directing molecule (e.g., polymer). A single disulfide Cys58-Cys105 appears essential for activity. The Cys125 residue appears not to be required for some uses and provides an additional functional group for derivatization. The three dimensional crystal structure of IL-2 has been solved to 3 angstrom resolution. Most of the secondary structure is alpha helical in nature. Three of the alpha helices (residues 11-19; residues 33-56; and residues 107-113) appear to be important for IL-2 binding and activity. Five of the twelve free amines are located in these helices, and, preferably, derivatization of IL-2 is not conducted via these amines. IL-2 is normally purified by mono-S and reverse phase high pressure liquid chromatography (RP). Elution conditions for RP (60% acetonitrile, pH 2-3) suggest a very stable or, at least, an elastic molecule. Consequently, IL-2 appears to be amenable to a number of conjugation techniques.

IL-2-biotin conjugates may be formed using biotinamidocaproate N-hydroxysuccinimide ester (commercially available from Sigma Chemical Co.). The reaction of IL-2 and biotinamidocaproate N-hydroxysuccinimde ester is conducted at room temperature for 0.5 hours in a 0.1M sodium borate buffer, pH 8.0-8.5, containing 0.1% sodium dodecyl sulfate to keep IL-2 in solution. When the IL-2 concentration is 5-10 mg/ml of reaction buffer, biotin is incorporated at 75-80% when imidate ester, dissolved in DMSO in a volume no greater than 5-10% of the total reaction volume, is added at a 2-4 biotin:IL-2 molar ratio. The product conjugate contains 1.5-3 biotins per IL-2 molecule. Biotin incorporation is assessed using the 2-(4-hydroxyazobenzene)benzoic acid (HABA displacement assay using pronase digested biotinylated IL-2.

Polymer-IL-2-biotin conjugates may be formed using commercially available biotinylated, lysine-derivatized dextran polymer (Sigma Chemical Co.) that is activated by reacting a lysine residue thereof with the bifunctional reagent SMCC, under conditions analogous to the biotinylation of IL-2 set forth above (e.g., molar ratio and the like). The SMCC-derivatized dextran polymer now contains the reactive maleimide functional group available for conjugation with the cysteine sulfhydryl moiety of IL-2 at pH 5-7 in acetate/phosphate buffer.

IL-2 receptor-bearing cells include activated T-cells, normal T-cells, activated B-cells, NK cells, LAK cells and thymocytes. High, intermediate and low affinity receptors for IL-2 exist. However, only about 10% of the receptors for IL-2 appear to be high affinity receptors (K_{d} approximately 10⁻¹¹M).

In the practice of a two-step pretargeting aspect of the present invention, IL-2 may be delivered to target cells as follows:
administering to the recipient a first conjugate comprising a targeting moiety and a member of a ligand-anti-ligand binding pair, wherein the first conjugate localizes to a target site;
optionally administering to the recipient a clearing agent capable of directing the clearance of circulating conjugate from the recipient or optionally treating the recipient with a clearing device or an alternative clearing procedure to substantially remove circulating conjugate from the recipient; and
administering to the recipient a second conjugate comprising an anti-tumor agent, such as IL-2, and a ligand/anti-ligand binding pair member, wherein the second conjugate binding pair member is complementary to that of the first conjugate.

One alternative to the optional clearance step set forth above is simply to allow an amount of time to pass that is sufficient to permit the recipient's native clearance mechanisms to substantially remove circulating conjugate.

Concentrations of IL-2 effective in maintaining activated tumor infiltrating lymphocytes (TIL) and for other forms of anti-tumor therapy have also been found to be toxic to the recipient. Localization of IL-2 to the tumor microenvironment allows localized activation of effector cells to poorly immunogenic tumor antigens. Effector cells of the cytotoxic T-lymphocyte lineage and T-helper lineage are induced to recognize tumor antigens and clonally expand to seek out tumor metastases at other locations.

Furthermore, B cells may be induced to secrete antibody specifically recognizing tumor antigens.

These B cells mature into IgG secretory cells and memory cells and continue to expand when tumor antigens are detected at other sites. In addition, natural killer cells are candidates for anti-tumor immunity, because such cells are also activated by IL-2. Studies in SCID-beige mice deficient in T and B cells but NK competent showed that such mice were effective hosts in rejecting an IL-2 transfected tumor cell line. See Alosco et al., Cancer Immunol. Immunother., 36: 364-372, 1993.

Tumor necrosis factors (TNFs) have been isolated from a variety of mammalian species. For example, human, murine, rabbit and guinea pig exhibit at least alpha and beta forms of TNF. In humans, TNF-alpha exhibits a molecular weight of 45 (gel filtration) and 17 (SDS-PAGE), an isoelectric point of 5.6, no glycosylation, protease sensitivity, 2 cysteine residues, 157 amino acids and a terminal valine residue. TNF-beta exhibits a molecular weight of 65 (gel filtration) and 25 (SDS-PAGE), an isoelectric point of 5.8, glycosylation, protease resistance, no cysteine residues, 171 amino acids, and a terminal leucine residue. The serum half-lives of TNF-alpha and TNF-beta are approximately 10 to 15 minutes.

TNF-alpha and TNF-beta exhibit free primary amines for conjugation to the ligand biotin, for example, or to a functional group of a biodistribution directing molecule. TNF-biotin conjugates may be formed as follows: Biotinamidocaproate N-hydroxysuccinimidate (dissolved in a small volume of DMSO) is offered at a 4-8 biotin:TNF molar ratio with respect to TNF (dissolved in 0.1M borate buffer, pH 8.0-8.5, at a protein concentration of 0.5 mg/ml). After 2 hours at room temperature TNF has incorporated 1 biotin per TNF molecule as assessed by the HABA assay.

Polymer-TNF-biotin conjugates may be formed using commercially available biotinylated, lysine-derivatized dextran polymer (10 kD-70 kD glucan polymer supplied by Sigma Chemical Co.) that is reacted with N-succinimidyl-3-(2-pyridyl-dithio)propionate (SPDP), under conditions analogous to the biotinylation of TNF as described above to give rise to a pyridyl dithio-derivatized polymer. TNF is derivatized by a lysine residue thereof with the bifunctional reagent SMCC, under conditions analogous to the biotinylation of TNF set forth above to give rise to maleimidyl-derivatized TNF. To the pyridyl dithio polymer, dithiothreitol (DTT) is added in an oxygen free environment at a 2:1 DTT:polymer molar ratio to generate a free sulfhydryl moiety on the polymer. After purification of the derivatized polymer in the oxygen free environment, the maleimidyl-derivatized TNF is added, wherein the maleimidyl group reacts with the sulfhydryl moiety of the derivatized polymer to form the product conjugate.

TNF receptor-bearing cells include adipocytes, myotubes, cervical carcinoma, fetal lung, bladder carcinoma, histocytic leukemia, erthroleukemia, promyelocytic leukemia, epidermoid carcinoma, cervical carcinoma, T lymphoma, human lymphocytes, lymphoblastic leukemia (two receptors), monocytic leukemia, foreskin fibroblast, connective tissue (two receptors), murine macrophage, and bovine endothelium. High, intermediate and low affinity receptors for TNF exist.

TNF-alpha and TNF-beta have been shown to exhibit different biological effects on certain target cells, including endothelial cells (production of IL-1), myeloid cells (clonogenic survival), fibroblasts and macrophages (production of macrophage colony-stimulating factor), neutrophils (activation of neutrophils), osteoblasts (proliferation, release of Ca⁺² collagen degradatioh), vascular smooth muscle cells (interferon-gamma-dependent expression of human lymphocyte antigen-DR) , T-cell hybridoma (MHC-I cell expression), and B lymphocytes (growth factor).

In the practice of a two-step pretargeting aspect of the present invention, TNF may be delivered to target cells essentially in the manner described above for IL-2.

TNF itself is cytotoxic to a narrow spectrum of tumor cells; however, this cytokine exhibits a broad range of immunologic modulating activities. One such activity is activation of tumor infiltrating macrophages or monocytes, thereby rendering the macrophages tumoricidal. One theory regarding the mechanism of TNF in this regard suggests that TNF stimulates monocytes to progress to macrophages which are, in turn, stimulated by TNF to release cytotoxic factors (e.g., oxidative burst or protease secretion or cytokine release). TNF release by activated macrophages can maintain or induce tumoricidal activity through an autocrine mechanism. Additional activation of monocytes or macrophages by other cytokines (e.g., gamma-interferon) may be employed to enhance the cytotoxic effect.

For example, membrane preparations from gamma-interferon-activated monocytes are cytotoxic to K562 (erythroleukemia cell line) or WEHI164 (murine fibrosarcoma cell line) target cells. Pretreatment of monocytes with recombinant TNF-alpha for 1 hour followed by treatment of medium alone or gamma-interferon led to increased killing of the aforementioned tumor cell types by the TNF-alpha/gamma-interferon-treated membrane preparations of monocytes. See, for example, Peck et al., Cellular Immunol., 132: 308, 1991).

Burrows et al., Proc. Natl. Acad. Sci. USA, 90: 8996-9000, 1993, discuss the concept of vascular targeting. Tumor target vascular endothelial cells are accessible to circulating agents. Burrows et al. transfected a neuroblastoma cell line with the murine INF-gamma gene. These transfected cells as well as their non-transfected counterparts were grown subcutaneously in BALB/c nu/nu mice. The transfected cells secreted INF-gamma, which induced expression of class II antigens of the major histocompatibility complex by capillary and venular endothelial cells within the tumor mass. An immunotoxin targeted to such MHC class II antigens was then administered and rapid accretion to tumor and tumor regressions were observed. Relapses were observed 7-10 days after treatment and were attributed to surviving tumor cells that derived nutrition from the extratumoral blood supply.

The pretargeting approach may be employed in conducting vascular targeting diagnostic or therapeutic protocols. For example, a suitable two-step pretargeting method useful in practicing vascular targeting includes the following:
administering to the recipient a first conjugate comprising a targeting moiety specific for tumor endothelial cells and INF-gamma, wherein the first conjugate localizes to a target site and the INF-gamma induces expression of MHC class II antigens by tumor endothelial cells;
optionally administering to the recipient a clearing agent capable of directing the clearance of circulating conjugate from the recipient or optionally treating the recipient with a clearing device or an alternative clearing procedure to substantially remove circulating conjugate from the recipient; and
administering to the recipient a second conjugate comprising an anti-tumor agent, such as a toxin, a radionuclide, an anti-tumor agent or the like, and a targeting agent specific for MHC class II antigens.

An alternative two-step pretargeting method useful in vascular targeting is as follows:
administering to the recipient a first conjugate comprising a targeting moiety specific for tumor endothelial cells and a member of a ligand-anti-ligand binding pair, wherein the first conjugate localizes to a target site;
optionally administering to the recipient a clearing agent capable of directing the clearance of circulating conjugate from the recipient or optionally treating the recipient with a clearing device or an alternative clearing procedure to substantially remove circulating conjugate from the recipient; and
administering to the recipient a second conjugate comprising an anti-tumor agent, such as a toxin, a radionuclide or an anti-tumor agent, and a ligand/anti-ligand binding pair member, wherein the second conjugate binding pair member is complementary to that of the first conjugate.
This latter protocol offers the advantages of not relying on tumor endothelial cells to express the antigen recognized by the active agent-bearing conjugate and of not exposing the recipient to systemically administered INF-gamma.

One alternative to the optional clearance step set forth above is simply to allow an amount of time to pass that is sufficient to permit the recipient's native clearance mechanisms to substantially remove circulating conjugate.

Also, an optional additional step is the administration of a conjugate incorporating a targeting moiety specific for tumor cells and a cytotoxic active agent. Alternatively, administration of a conjugate comprising a targeting moiety specific for tumor cells and the member of the ligand-anti-ligand binding pair incorporated in the first conjugate, wherein this conjugate localizes to a target site. In this manner, tumor cells that receive nutrition from the extratumoral vasculature can be addressed.

A protocol, such as administration of streptavidin-targeting moiety conjugate followed by administration of biotinylated cytokine, is also contemplated by the present invention. Such pretargeting of anti-ligand serves to improve the performance of cytokine therapeutics by increasing the amount of cytokine localized to target cells.

Streptavidin-antibody conjugates generally exhibit pharmacokinetics similar to the native antibody and localize well to target cells, depending upon their construction. Biotinylated cytokines retain a short *in vivo* half-life; however, cytokine may be localized to the target as a result of the affinity of biotin for avidin. In addition, biotin-avidin experience a pH-dependent dissociation which occurs at a slow rate, thereby permitting a relatively constant, sustained release of cytokine at the target site over time. Also, cytokines complexed to target cells through biotin-avidin association are available for extraction and internalization by cells involved in cellular-mediated cytotoxicity.

A pre-formed antibody-streptavidin-biotin-cytokine preparation may also be employed in the practice of these methods of the present invention. In addition, a three-step protocol of the present invention may also be employed to deliver a cytokine, such as IL-2, to a target site.

It is another object of the invention to increase the utilization of cytokine (ligand or anti-ligand) conjugates at targeted sites, e.g., a tumor. More particularly, it is an object of the invention to increase the utilization of TNF-biotin/(streptavidin or avidin)-antibody conjugate system as a delivery system for delivery of cytokines to tumor cells. This is accomplished by providing for the site specific release of biologically active, "free" cytokine, e.g., TNF at the targeted site.

This is accomplished by the administration of a complex of a targeting moiety - (ligand or anti-ligand) conjugate, and a cytokine (ligand or anti-ligand) conjugate or moieties which provide for formation of such a complex. The prototypical embodiment comprises the administration of a complex of NR-LU-10/streptavidin precomplexed with biotinylated TNF. This "premade" fusion construct is an effective means for site-specifically delivering a cytokine, e.g., TNF, to a tumor site. Moreover, it is believed that this may decrease systemic toxicity associated with TNF.

In this regard, the utilization of cytokines, including tumor necrosis factor (TNF), has been limited by both their systemic toxicity and the lack of effective targeting to desired sites of action. This application describes methods which utilize pretargeting to effect the site-specific delivery of cytokines to target sites. Another methodology which can be applied to both pretargeted (separately administered) and precomplexed (Ab-StrAv(Bt-cytokine)) should provide for the release of "free," more biologically active cytokine, e.g., TNF, at the target site, following cytokine localization at the target site by either of the aforementioned technologies. TNF is a protein synthesized and secreted by mononuclear phagocytes in response to stimulation by bacterial endotoxin and other agents. It has been shown to produce hemorrhagic necrosis of tumors *in vivo*. The delivery of TNF to target sites is plagued by its short half-life (11-30 min. in humans) and its toxic effects on the cardiovascular system, where hypotension is dose limiting. This hypotension is most likely a result of TNF-mediated release of nitric oxide by vascular endothelial cells, as is seen in septic shock. Pretargeting has been shown to be an effective methodology for "trapping" molecules with a relatively short serum half-life at the tumor, allowing their accretion to high concentrations within a short time. These molecules are then retained for long periods of time due to slow off-rate and/or diffusion of the antibody-streptavidin conjugate from the tumor.

Therefore, since TNF is toxic due to its effects on the vasculature, the same toxicity might be expected for biotinylated TNF (TNF-Bt) in a pretargeting approach. This could be addressed by a very slow infusion of this material, but one would potentially still be exposing the vasculature to some concentration of TNF. Accordingly, it is an object of the invention to "mask" this toxicity by precomplexing TNF-Bt with an antibody/StrAv, therefore allowing the accretion at the target to be antibody-dependent, in a pharmacokinetic sense. Assuming that this construct is less (or non-) toxic, there is a possibility that it might be less efficacious against target cells. If this is the case, to release "free", biologically active TNF-Bt at the target, one could allow TNF-Bt conjugate to accrete to peak levels at the target site, then administer systemically either biotin or a non-toxic small molecule biotinylated species which will act to disrupt the equilibrium of TNF-Bt which is coming off the Ab/StrAv, replacing the TNF-Bt as it comes off the Ab-StrAv, and allowing it to build up a concentration of "free" TNF-Bt. Since TNF is a protein of approximately 16 kD molecular weight, it should not diffuse away rapidly from the tumor, therefore allowing an effective local concentration to be achieved.

The idea for this embodiment of the invention was in vivo experimental data which demonstrated that a non-covalent conjugate of StrAv and biotinylated NR-LU-10 dissociated at a vary rapid rate (less than a day) in the presence of a high concentration of free biotin, but dissociated much more slowly in the absence of biotin. This result provided evidence that by administering a "pulse" of biotin should allow an "offloading" of a therapeutic species following target localization.

It is believed that free biotin administration should yield a corresponding increase in serum biotin. levels to facilitate dissociation of free cytokine. However, assuming that enough concentrations of biotin. cannot be attained by biotin administration, (because of tight *in vivo* biotin regulation) then administering a biotinylated species such as a small molecule, protein or polymer, with appropriate pharmacokinetics, should obviate this potential problem.

Thus, based on the foregoing, this embodiment of the invention essentially, comprises exploiting the non-covalent nature of TNF-Bt a cytokine-ligand or: anti-ligand e.g.interaction at the target site, whether it was pretargeted or precomplexed, and to facilitate its release by competing it off with ligand or anti-ligand, e.g. biotin. Additional improvement of this embodiment further include the use of appropriate low-affinity analogs of the ligand or anti-ligand biotin to provide for the release of free cytokine, e.g. TNF.

Other anti-tumor agents that may be delivered in accordance with the pretargeting techniques of the present invention are selectins, including L-selectin, P-selectin and E-selectin. The presence of cytokines stimulates cells, such as endothelial cells, to express selectins on the surfaces thereof. Selectins bind to white blood cells and aid in delivering white blood cells where they are needed. Consequently, a protocol, such as administration of streptavidin- or avidin-targeting moiety conjugate followed by administration of biotinylated selectins, is also contemplated by the present invention. Such pretargeting of anti-ligand serves to improve the performance of selectin therapeutics by increasing the amount of selectin localized to target cells. In this manner, the necessity of cytokine induction of selectin expression is obviated by the localization and retention of selectin at a target cell population.

While the majority of the exemplary pretargeting protocols set forth above have been directed to the treatment of cancer, pretargeting protocols may be employed in the diagnosis or treatment of other conditions. Exemplary additional conditions are discussed below.

For example, active agents that are cytotoxic to activated cytotoxic T-cells, such as trichothecenes (e.g., Roridin A, Verrucarin A, anguidine, scirpenetriol and the like) as well as agents that decrease helper T-cell activity or helper T-cell number, such as TGF-beta, or increase Ts activity, and the like will facilitate the treatment of autoimmune diseases and transplantation rejection prevention. Tissue lesions in autoimmune disease are caused by the direct action of activated cytotoxic T-cells. Tissue transplantation is also, in part, mediated by the action of activated T-cells that bind to the cells of the new tissue and lyse them. Active agents that selectively de-activate or selectively kill activated cytotoxic T-cells can be employed as active agents in pretargeting protocols of the present invention directed at the treatment of autoimmune disease or the prevention of tissue rejection. Such active agents may also be administered in combination with a second active agent that disrupts antibody (produced by B cells with the assistance of helper T-cells and IL-2)-complement mediated cell lysis associated with graft rejection.

Targeting to the target tissues may be accomplished via antibodies directed to the IL-2 receptor such as anti-TAC, anti-IL-2 receptor antibodies (e.g., anti-P-55 receptor and anti-P-75 receptor) or by IL-2 itself or the like. IL-2 is rapidly internalized upon binding to its receptor and is otherwise primarily excreted via the renal pathway. Consequently, IL-2 would be a less desirable targeting moiety for pretargeting, as it may not remain on the surface of target cells long enough to allow completion of the protocol.

For example, delivery of active agents, such as interferon-gamma, tumor necrosis factor-alpha or a combination thereof, to monocytes or macrophages.or tumor cells via pretargeting protocols will facilitate the treatment of cancer through the mechanism of activating the monocytes to cytotoxic macrophages. as Activated macrophages excrete toxic moieties such as enzymes, lysozyme cathepsins and hydrolases. Tumor necrosis factor-alpha acts via activation of cytotoxic macrophages, direct tumor cell killing or rendering the tumor cells more susceptible to effector cell-mediated cytotoxicity. A combination of the two active agents serves to optimally prime macrophage tumoricidal activity. Targeting to the target tissues may be accomplished via antibodies directed to tumor specific antigens, such that the active agents are presented to the infiltrating monocyte population in a localized environment.

For example, delivery of active agents, such as interleukin-2, to cytotoxic T-cells via pretargeting protocols will facilitate the treatment of cancer through the mechanism of activating tumor infiltrating lymphocytes (TIL). Killing by cytotoxic T-cells is believed to be mediated by release of pore-forming moieties that insert into the membrane of the target cell and facilitate target cell lysis. Targeting to the target tissues may be accomplished via antibodies directed to tumor specific antigens.

For example, delivery of active agents, such as granulocyte monocyte colony stimulating factor (GM-CSF), to macrophages and polymorphonuclear neutrophils via pretargeting protocols will facilitate the treatment of cancer through the mechanism of activating infiltrating monocytes and polymorphonuclear leukocytes (PMNs). Killing by monocytes and polymorphonuclear leukocytes is the result of cytotoxic enzymes released by activated forms of these cells. Targeting to the target tissues may be accomplished via antibodies directed to tumor specific antigens.

For example, delivery of active agents, such as transforming growth factor-beta (TGF-beta), to pancreatic tissue via pretargeting protocols will facilitate the treatment of insulin-dependent diabetes mellitus through the mechanism of inhibition of cytotoxic T-cell maturation and inhibition of T-cell proliferation. Delivery to pancreatic tissue may be accomplished, for example, an antibody or other targeting moiety which recognizes an antigen present on islet cells. Killing by T-cells is the result of the release of pore-forming moieties by activated forms of these cells. Targeting to the target tissues may be accomplished via antibodies directed to the pancreas.

TGF-beta may also be employed in the treatment of inflammatory disease using pretargeting protocols of the present invention. Exemplary chronic inflammatory diseases which may be so treated are rheumatoid arthritis, thyroid disease in Hashimoto's thyroiditis, tuberculoid granuloma and the like. TGF-beta is delivered to target by an antibody or other targeting moiety which recognizes fibroblast activation protein or antigen 19 on stimulated stromal fibroblasts. See, for example, Rettig et al., Cancer. Res., 53: 3327, 1993. In this manner, TGF-beta may be used to inhibit chronic inflammation by targeting stromal fibroblasts activated by peptide mediators and proteolytic enzymes.

An additional aspect of the present invention is directed to the use of targeting moieties that are monoclonal antibodies or fragments thereof that localize to an antigen that is recognized by the antibody NR-LU-10. Such monoclonal antibodies or fragments may be murine or of other non-human mammalian origin, chimeric, humanized or human.

NR-LU-10 is a 150 kilodalton molecular weight IgG2b monoclonal antibody that recognizes an approximately 40 kilodalton glycoprotein antigen expressed on most carcinomas. *in vivo* studies in mice using an antibody specific for the NR-LU-10 antigen revealed that such antibody was not rapidly internalized, which would have prevented localization of the subsequently administered active-agent-containing conjugate to the target site.

NR-LU-10 is a well characterized pancarcinoma antibody that has been safely administered to over 565 patients in human clinical trials. The hybridoma secreting NR-LU-10 was developed by fusing mouse splenocytes immunized with intact cells of a human small cell lung carcinoma with P3 x 63/Ag8UI murine myeloma cells. After establishing a seed lot, the hybridoma was grown via *in vitro* cell culture methods, purified and verified for purity and sterility .

Radioimmunoassays, immunoprecipitation and Fluorescence-Activated Cell Sorter (FACS) analysis were used to obtain reactivity profiles of NR-LU-10. The NR-LU-10 target antigen was present on either fixed cultured cells or in detergent extracts of various types of cancer cells . For example, the NR-LU-10 antigen is found in small cell lung, non-small cell lung, colon, breast, renal, ovarian, pancreatic, and other carcinoma tissues. Tumor reactivity of the NR-LU-10 antibody is set forth in Table A, while NR-LU-10 reactivity with normal tissues is set forth in Table B. The values in Table B are obtained as described below. Positive NR-LU-10 tissue reactivity indicates NR-LU-10 antigen expression by such tissues. The NR-LU-10 antigen has been further described by Varki et al., "Antigens Associated with a Human Lung Adenocarcinoma Defined by Monoclonal Antibodies," Cancer Research, 44: 681-687, 1984, and Okabe et al., "Monoclonal Antibodies to Surface Antigens of Small Cell Carcinoma of the Lung," Cancer Research, 44: 5273-5278, 1984.

The tissue specimens were scored in accordance with three reactivity parameters: (1) the intensity of the reaction; (2) the uniformity of the reaction within the cell type; and (3) the percentage of cells reactive with the antibody. These three values are combined into a single weighted comparative value between 0 and 500, with 500 being the most intense reactivity. This comparative value facilitates comparison of different tissues. Table B includes a summary reactivity value, the number of tissue samples examined and the number of samples that reacted positively with NR-LU-10.

Methods for preparing antibodies that bind to epitopes of the NR-LU-10 antigen are described in U.S. Patent No. 5,084,396. Briefly, such antibodies may be prepared by the following procedure:
- absorbing a first monoclonal antibody directed against a first epitope of a polyvalent antigen onto an inert, insoluble matrix capable of binding immunoglobulin, thereby forming an immunosorbent;
- combining the immunosorbent with an extract containing polyvalent NR-LU-10 antigen, forming an insolubilized immune complex wherein the first epitope is masked by the first monoclonal antibody;
- immunizing an animal with the insolubilized immune complex;
- fusing spleen cells from the immunized animal to myeloma cells to form a hybridoma capable of producing a second monoclonal antibody directed against a second epitope of the polyvalent antigen;
- culturing the hybridoma to produce the second monoclonal antibody; and
- collecting the second monoclonal antibody as a product of the hybridoma.
Consequently, monoclonal antibodies NR-LU-01, NR-LU-02 and NR-LU-03, prepared in accordance with the procedures described in the aforementioned patent, are exemplary targeting moieties useful in this aspect of the present invention.

Additional antibodies reactive with the NR-LU-10 antigen may also be prepared by standard hybridoma production and screening techniques. Any hybridoma clones so produced and identified may be further screened as described above to verify antigen and tissue reactivity.

**Table A**

| **TUMOR REACTIVTTY OR ANTIBODY NR-LU-10** | | | | | | | |
|---|---|---|---|---|---|---|---|
| Organ/Cell Type Tumor | #Pos/ Exam | Intensity^{a}Avg. Range | | Percent^{b} Avg. Range | | Uniformity^{c} Avg. Range | |
| Pancreas Carcinoma | 6/6 | 3 | 3 | 100 | 100 | 2.3 | 2-3 |
| Prostate Carcinoma | 9/9 | 2.8 | 2-3 | 95 | 80-100 | 2 | 1-3 |
| Lung Adenocarcinoma | 8/8 | 3 | 3 | 100 | 100 | 2.2 | 1-3 |
| Lung Small Cell Carcinoma | 2/2 | 3 | 3 | 100 | 100 | 2 | 2 |
| Lung Squamous Cell Carcinoma | 8/8 | 2.3 | 2-3 | 73 | 5-100 | 1.8 | 1-3 |
| Renal Carcinoma | 8/9 | 2.2 | 2-3 | 83 | 75-100 | 1 | 1 |
| Breast Adenocarcinoma | 23/23 | 2.9 | 2-3 | 97 | 75-100 | 2.8 | 1-3 |
| Colon Carcinoma | 12/12 | 2.9 | 2-3 | 98 | 95-100 | 2.9 | 2-3 |
| Malignant Melanoma Ocular | 0/2 | 0 | 0 | 0 | 0 | 0 | 0 |
| Malignant Melanoma | 0/11 | 0 | 0 | 0 | 0 | 0 | 0 |
| Ovarian Carcinoma | 35/35 | 2.9 | 2-3 | 200 | 100 | 2.2 | 1-3 |
| Undifferentiated Carcinoma | 1/1 | 2 | 2 | 90 | 90 | 2 | 2 |
| Osteosarcoma | 1/1 | 2 | 2 | 20 | 20 | 1 | 1 |
| Synovial Sarcoma | 0/1 | 0 | 0 | 0 | 0 | 0 | 0 |
| Lymphoma | 0/2 | 0 | 0 | 0 | 0 | 0 | 0 |
| Liposarcoma | 0/2 | 0 | 0 | 0 | 0 | 0 | 0 |
| Uterine Leiomyosarcoma | 0/1 | 0 | 0 | 0 | 0 | 0 | 0 |

| | | | | | | | |
|---|---|---|---|---|---|---|---|
| ^{a} Rated from 0-3, with 3 representing highest intensity. | | | | | | | |
| ^{b} Percentage of cells stained within the examined tissue section. | | | | | | | |
| ^{c} Rates from 0-3, with 3 representing highest uniformity. | | | | | | | |

**Table B**

| **Organ/Cell Type** | **# Pos/Exam** | **Summary Reactivity** |
|---|---|---|
| Adenoid | | |
| Epithelium | 3 | 433 |
| Lymphoid Follicle-Central | 0/3 | 0 |
| Lymphoid Follicle-Peripheral | 2/2 | 400 |

| Adipose Tissue | | |
|---|---|---|
| Fat Cells | 0/3 | 0 |

| Adrenal | | |
|---|---|---|
| Zona Fasciculata Cortex | 0/3 | 0 |
| Zona Glomerulosa Cortex | 0/3 | 0 |
| Zona Reticularis Cortex | 0/3 | 0 |
| Medulla | 0/3 | 0 |

| Aorta | | |
|---|---|---|
| Endothelium | 0/3 | 0 |
| Elastic Interna | 0/3 | 0 |
| Tunica Adventitia | 0/3 | 0 |
| Tunica Media | 0/3 | 0 |

| Brain-Cerebellum | | |
|---|---|---|
| Axons, Myelinated | 0/3 | 0 |
| Microglia | 0/3 | 0 |
| Neurons | 0/3 | 0 |
| Purkenje's Cells | 0/3 | 0 |

| Brain-Cerebrum | | |
|---|---|---|
| Axons, Myelinated | 0/3 | 0 |
| Microglia | 0/3 | 0 |
| Neurons | 0/3 | 0 |

| Brain-Midbrain | | |
|---|---|---|
| Axons, Myelinated | 0/3 | 0 |
| Microglia | 0/3 | 0 |
| Neurons | 0/3 | 0 |

| Colon | | |
|---|---|---|
| Mucosal Epithelium | 3/3 | 500 |
| Muscularis Externa | 0/3 | 0 |
| Muscularis Mucosa | 0/3 | 0 |
| Nerve Ganglia | 0/3 | 0 |
| Serosa | 0/1 | 0 |

| Duodenum | | |
|---|---|---|
| Mucosal Epithelium | 3/3 | 500 |
| Muscularis Mucosa | 0/3 | 0 |

| Epididymis | | |
|---|---|---|
| Epithelium | 3/3 | 419 |
| Smooth Muscle | 0/3 | 0 |
| Spermatozoa | 0/1 | 0 |

| Esophagus | | |
|---|---|---|
| Epithelium | 3/3 | 86 |
| Mucosal Gland | 2/2 | 450 |
| Smooth Muscle | 0/3 | 0 |

| Gall Bladder | | |
|---|---|---|
| Mucosal Epithelium | 0/3 | 467 |
| Smooth Muscle | 0/3 | 0 |

| Heart | | |
|---|---|---|
| Myocardium | 0/3 | 0 |
| Serosa | 0/1 | 0 |

| Ileum | | |
|---|---|---|
| Lymph Node | 0/2 | 0 |
| Mucosal Epithelium | 0/2 | 0 |
| Muscularis Externa | 0/1 | 0 |
| Muscularis Mucosa | 0/2 | 0 |
| Nerve Ganglia | 0/1 | 0 |
| Serosa | 0/1 | 0 |

| Jejunum | | |
|---|---|---|
| Lymph Node | 0/1 | 0 |
| Mucosal Epithelium | 2/2 | 400 |
| Muscularis Externa | 0/2 | 0 |
| Muscularis Mucosa | 0/2 | 0 |
| Nerve Ganglia | 0/2 | 0 |
| Serosa | 0/1 | 0 |

| Kidney | | |
|---|---|---|
| Collecting Tubules | 2/3 | 160 |
| Distal Convoluted Tubules | 3/3 | 500 |
| Glomerular Eipthelium | 0/3 | 0 |
| Mesangial | 0/3 | 0 |
| Proximal Convoluted Tubules | 3/3 | 500 |

| Liver | | |
|---|---|---|
| Bile Duct | 3/3 | 500 |
| Central Lobular Hepatocyte | 1/3 | 4 |
| Periportal Hepatocyte | 1/3 | 40 |
| Kupffer Cells | 0/3 | 0 |

| Lung | | |
|---|---|---|
| Alveolar Macrophage | 0/3 | 0 |
| Bronchial Epithelium | 0/2 | 0 |
| Bronchial Smooth Muscle | 0/2 | 0 |
| Pneumocyte Type I | 3/3 | 354 |
| Pneumocyte Type II | 3/3 | 387 |

| Lymph Node | | |
|---|---|---|
| Lymphoid Follicle-Central | 0/3 | 0 |
| Lymphoid Follicle-Peripheral | 0/3 | 0 |

| Mammary Gland | | |
|---|---|---|
| Alveolar Epithelium | 3/3 | 500 |
| Duct Epithelium | 3/3 | 500 |
| Myoepithelium | 0/3 | 0 |

| Muscle Skeletal | | |
|---|---|---|
| Muscle Fiber | 0/3 | 0 |

| Nerve | | |
|---|---|---|
| Axon, Myelinated | 0/2 | 0 |
| Endoneurium | 0/2 | 0 |
| Neurolemma | 0/2 | 0 |
| Neuron | 0/2 | 0 |
| Perineurium | 0/2 | 0 |

| Ovary | | |
|---|---|---|
| Corpus Luteum | 0/3 | 0 |
| Eipthelium | 1/1 | 270 |
| Granulosa | 1/3 | 400 |
| Serosa | 0/3 | 0 |
| Theca | 0/3 | 0 |

| Oviduct | | |
|---|---|---|
| Epithelium | 1/1 | 500 |
| Smooth Muscle | 0/3 | 0 |

| Pancreas | | |
|---|---|---|
| Acinar Cell | 3/3 | 500 |
| Duct Epthelium | 3/3 | 500 |
| Islet Cell | 3/3 | 500 |

| Peritoneum | | |
|---|---|---|
| Mesothelium | 0/1 | 0 |

| Pituitary | | |
|---|---|---|
| Adenohypophysis | 2/2 | 500 |
| Neurohypophysis | 0/2 | 0 |

| Placenta | | |
|---|---|---|
| Trophoblasts | 0/3 | 0 |

| Prostate | | |
|---|---|---|
| Concretions | 0/3 | 0 |
| Glandular Epithelium | 3/3 | 400 |
| Smooth Muscle | 0/3 | 0 |

| Rectum | | |
|---|---|---|
| Lymph Node | 0/2 | 0 |
| Mucosal Epithelium | 0/2 | 0 |
| Muscularis Externa | 0/1 | 0 |
| Muscularis Mucosa | 0/3 | 0 |
| Nerve Ganglia | 0/3 | 0 |

| Salivary Gland | | |
|---|---|---|
| Acinar Epithelium | 3/3 | 500 |
| Duct Epithelium | 3/3 | 500 |

| Skin | | |
|---|---|---|
| Apocrine Glands | 3/3 | 280 |
| Basal Layer | 3/3 | 33 |
| Epithelium | 1/3 | 10 |
| Follicle | 1/1 | 190 |
| Stratum Corneum | 0/3 | 0 |

| Spinal Cord | | |
|---|---|---|
| Axons, Myelinated | 0/2 | 0 |
| Microglial | 0/2 | 0 |
| Neurons | 0/2 | 0 |

| Spleen | | |
|---|---|---|
| Lymphoid Follicle-Central | 0/3 | 0 |
| Lymphoid Follicle-Peripheral | 0/3 | 0 |
| Trabecular Smooth Muscle | 0/3 | 0 |

| Stomach | | |
|---|---|---|
| Chief Cells | 3/3 | 290 |
| Mucosal Epithelium | 3/3 | 367 |
| Muscularis Mucosa/Externa | 0/3 | 0 |
| Parietal Cells | 3/3 | 290 |
| Smooth Muscle | 0/3 | 0 |

| Stromal Tissue | | |
|---|---|---|
| Adipose | 0/63 | 0 |
| Arteriolar Smooth Muscle | 0/120 | 0 |
| Endothelium | 0/120 | 0 |
| Fibrous Connective Tissue | 0/120 | 0 |
| Macrophages | 0/117 | 0 |
| Mast Cells/Eosinophils | 0/86 | 0 |

| Testis | | |
|---|---|---|
| Interstitial Cells | 0/3 | 0 |
| Sertoli Cells | 3/3 | 93 |

| Thymus | | |
|---|---|---|
| Hassal's Epithelium | 3/3 | 147 |
| Hassal's Keratin | 3/3 | 333 |
| Lymphoid Cortex | 0/3 | 0 |
| Lymphoid Medulla | 3/3 | 167 |

| Thyroid | | |
|---|---|---|
| C-Cells | 0/3 | 0 |
| Colloid | 0/3 | 0 |
| Follicular Epithelium | 3/3 | 500 |

| Tonsil | | |
|---|---|---|
| Epithelium | 1/3 | 500 |
| Lymphoid Follicle-Central | 0/3 | 0 |
| Lymphoid Follicle-Peripheral | 0/3 | 0 |
| Mucus Gland | 1/1 | 300 |
| Striated Muscle | 0/3 | 0 |

| Umbilical Cord | | |
|---|---|---|
| Epithelium | 0/3 | 0 |

| Urinary Bladder | | |
|---|---|---|
| Mucosal Epithelium | 3/3 | 433 |
| Serosa | 0/1 | 0 |
| Smooth Muscle | 0/3 | 0 |

| Uterus | | |
|---|---|---|
| Endometrial Epithelium | 3/3 | 500 |
| Endometrial Glands | 3/3 | 500 |
| Smooth Muscle | 0/3 | 0 |

| Vagina/Cervix | | |
|---|---|---|
| Epithelial Glands | 1/1 | 500 |
| Smooth Muscle | 0/2 | 0 |
| Squamous Epithelium | 1/1 | 200 |

### Example I

### Targeting Moiety-Anti-Ligand Conjugate for Two-Step Pretargeting In Vivo

### A. Preparation of SMCC-derivatized streptavidin.

31 mg (0.48 µmol) streptavidin was dissolved in 9.0 ml PBS to prepare a final solution at 3.5 mg/ml. The pH of the solution was adjusted to 8.5 by addition of 0.9 ml of 0.5 M borate buffer, pH 8.5. A DMSO solution of SMCC (3.5 mg/ml) was prepared, and 477 µl (4.8 µmol) of this solution was added dropwise to the vortexing protein solution. After 30 minutes of stirring, the solution was purified by G-25 (PD-10, Pharmacia, Piscataway, New Jersey) column chromatography to remove unreacted or hydrolyzed SMCC. The purified SMCC-derivatized streptavidin was isolated (28 mg, 1.67 mg/ml).

B. Preparation of DTT-reduced NR-LU-10. To 77 mg NR-LU-10 (0.42 µmol) in 15.0 ml PBS was added 1.5 ml of 0.5 M borate buffer, pH 8.5. A DTT solution, at 400 mg/ml (165 µl) was added to the protein solution. After stirring at room temperature for 30 minutes, the reduced antibody was purified by G-25 size exclusion chromatography. Purified DTT-reduced NR-LU-10 was obtained (74 mg, 2.17 mg/ml).

C. Conjugation of SMCC-streptavidin to DTT- reduced NR-LU-10. DTT-reduced NR-LU-10 (63 mg, 29 ml, 0.42 µmol) was diluted with 44.5 ml PBS. The solution of SMCC-streptavidin (28 mg, 17 ml, 0.42 µmol) was added rapidly to the stirring solution of NR-LU-10. Total protein concentration in the reaction mixture was 1.0 mg/ml. The progress of the reaction was monitored by HPLC (Zorbax® GF-250, available from MacMod). After approximately 45 minutes, the reaction was quenched by adding solid sodium tetrathionate to a final concentration of 5 mM.

D. Purification of conjugate. For small scale reactions, monosubstituted or disubstituted with regard to streptavidin conjugate was obtained using HPLC Zorbax (preparative) size exclusion chromatography. The desired monosubstituted or disubstituted conjugate product eluted at 14.0-14.5 min (3.0 ml/min flow rate), while unreacted NR-LU-10 eluted at 14.5-15 min and unreacted derivatized streptavidin eluted at 19-20 min.

For larger scale conjugation reactions, monosubstituted or disubstituted adduct is isolatable using DEAE ion exchange chromatography. After concentration of the crude conjugate mixture, free streptavidin was removed therefrom by eluting the column with 2.5% xylitol in sodium borate buffer, pH 8.6. The bound unreacted antibody and desired conjugate were then sequentially eluted from the column using an increasing salt gradient in 20 mM diethanolamine adjusted to pH 8.6 with sodium hydroxide.

E. Characterization of Conjugate.
1. HPLC size exclusion was conducted as described above with respect to small scale purification.
2. SDS-PAGE analysis was performed using 5% polyacrylamide gels under non-denaturing conditions. Conjugates to be evaluated were not boiled in sample buffer containing SDS to avoid dissociation of streptavidin into its 15 kD subunits. Two product bands were observed on the gel, which correspond to the mono- and di- substituted conjugates.
3. Immunoreactivity was assessed, for example, by competitive binding ELISA as compared to free antibody. Values obtained were within 10% of those for the free antibody.
4. Biotin binding capacity was assessed, for example, by titrating a known quantity of conjugate with p-[I-125]iodobenzoylbiocytin. Saturation of the biotin binding sites was observed upon addition of 4 equivalences of the labeled biocytin.
5. *in vivo* studies are useful to characterize the reaction product, which studies include, for example, serum clearance profiles, ability of the conjugate to target antigen-positive tumors, tumor retention of the conjugate over time and the ability of a biotinylated molecule to bind streptavidin conjugate at the tumor. These data facilitate determination that the synthesis resulted in the formation of a 1:1 streptavidin-NR-LU-10 whole antibody conjugate that exhibits blood clearance properties similar to native NR-LU-10 whole antibody, and tumor uptake and retention properties at least equal to native NR-LU-10.

For example, Figure 1 depicts the tumor uptake profile of the NR-LU-10-streptavidin conjugate (LU-10-StrAv) in comparison to a control profile of native NR-LU-10 whole antibody. LU-10-StrAv was radiolabeled on the streptavidin component only, giving a clear indication that LU-10-StrAv localizes to target cells as efficiently as NR-LU-10 whole antibody itself.

## Claims

1. Use of an active agent conjugate comprising a cytokine or growth factor active agent selected from the group consisting of cytokines, interleukins, transforming growth factor-beta, tumor necrosis factors, interferons, colony stimulating factors, vascular permeability factor and selectins; and a ligand/anti-ligand binding pair member, in the manufacture of a composition for use in a method of increasing highly toxic active agent localization at a target cell site within a mammalian recipient, which method comprises:
administering to the recipient a first conjugate comprising a targeting moiety and a member of a ligand-anti-ligand binding pair which is complementary to that of the active agent conjugate;
subsequently administering to the recipient the active agent conjugate.

2. Use according to claim 1 wherein the cytokine or growth factor active agent is selected from the group consisting of tumor necrosis factor, interleukin-2, transforming growth factor-beta, interleukin-4, interleukin-10, interferon-gamma, interferon-alpha, granulocyte macrophage-colony stimulating factor, and granulocyte-colony stimulating factor.

3. Use according to claim 1 or 2 in which the method further comprises administering to the recipient a clearing agent capable of directing circulating first conjugate to Ashwell receptors on hepatocytes, thereby decreasing the amount of circulating first conjugate prior to administering the active agent conjugate.

4. Use according to any preceding claim wherein the first conjugate comprises an anti-ligand and the active agent conjugate comprises a ligand, wherein preferably the anti-ligand is streptavidin and the ligand is biotin.

5. Use according to claim 4 in which the ligand is biotin and in which the method further comprises reducing the recipient's endogenous biotin level prior to, concurrently with or following administration of the first conjugate.

6. Use according to any one of claims 1 to 3 wherein the first conjugate comprises a ligand and the active agent conjugate comprises an anti-ligand, wherein preferably the ligand is biotin and the anti-ligand is streptavidin.

7. Use according to any one of claims 1 to 6 wherein the targeting moiety is a monoclonal antibody or an antigen-recognizing fragment thereof which is reactive with an antigen recognized by NR-LU-10 antibody.

8. A kit comprising the following pharmaceutical compositions for concurrent or sequential administration to a mammalian recipient:
a) a first composition comprising a first conjugate comprising a targeting moiety and a member of a ligand-anti-ligand binding pair; and
b) a second composition comprising an active agent conjugate comprising a cytokine or growth factor active agent selected from the group consisting of cytokines, interleukins, transforming growth factor-beta, tumor necrosis factors, interferons, colony stimulating factors, vascular permeability factor and selectins; and a ligand/antiligand binding pair member, wherein the active agent conjugate binding pair member is complementary to that of the first conjugate; and
c) optionally a third composition comprising a clearing agent capable of directing circulating first conjugate to Ashwell receptors on hepatocytes, thereby decreasing the amount of circulating first conjugate prior to administering the second conjugate.

9. A kit according to claim 8 wherein the cytokine or growth factor active agent in the second composition is selected from the group consisting of tumor necrosis factor, interleukin-2, transforming growth factor-beta, interleukin-4, interleukin-10, interferon-gamma, interferon-alpha, granulocyte macrophage-colony stimulating factor, and granulocyte-colony stimulating factor.

10. A kit according to claim 8 or 9 having the further feature(s) defined in claim 4 or claim 6 wherein when the first conjugate comprises an antiligand it is streptavidin and the ligand is biotin or when the first conjugate comprises a ligand it is biotin and the antiligand is streptavidin.

## Patentansprüche

1. Verwendung eines Wirkstoffkonjugats umfassend einen Cytokin- oder Wachstumsfaktor-Wirkstoff ausgewählt aus der Gruppe bestehend aus Cytokinen, Interleukinen, transformierendem Wachstumsfaktor-beta, Tumor-Nekrose-Faktoren, Interferonen, koloniestimulierenden Faktoren, vaskulärem Permeabilitätsfaktor und Selektinen und ein Ligand/Antiligand-Bindungspaar-Element bei der Herstellung einer Zusammensetzung zur Verwendung in einem Verfahren zum Erhöhen der Lokalisierung von hochtoxischem Wirkstoff an einer Target-Zellstelle in einem Säugetier-Empfänger, wobei das Verfahren umfasst:
Verabreichung eines ersten Konjugats umfassend eine Targeting-Gruppierung und ein Element eines Ligand/Antiligand-Bindungspaares, welches komplementär zu dem des Wirkstoffkonjugats ist, an den Empfänger,
anschließende Verabreichung des Wirkstoffkonjugats an den Empfänger.

2. Verwendung nach Anspruch 1, worin der Cytokin- oder Wachstumsfaktor-Wirkstoff ausgewählt ist aus der Gruppe bestehend aus Tumor-Nekrose-Faktor, Interleukin-2, transformierendem Wachstumsfaktor-beta, Interleukin-4, Interleukin-10, Interferon-gamma, Interferon-alpha, Granulozyten-Makrophagen-koloniestimulierendem Faktor und Granulozyten-koloniestimulierendem Faktor.

3. Verwendung nach Anspruch 1 oder 2, worin das Verfahren ferner umfasst das Verabreichen eines Clearing-Mittels an den Empfänger, das in der Lage ist, das zirkulierende erste Konjugat zu Ashwell-Rezeptoren auf Leberzellen zu leiten, wodurch die Menge an zirkulierendem erstem Konjugat vor der Verabreichung des Wirkstoffkonjugats verringert wird.

4. Verwendung nach irgendeinem vorhergehenden Anspruch, worin das erste Konjugat einen Antiliganden umfasst und das Wirkstoffkonjugat einen Liganden umfasst, wobei vorzugsweise der Antiligand Streptavidin und der Ligand Biotin ist.

5. Verwendung nach Anspruch 4, worin der Ligand Biotin ist und worin das Verfahren ferner die Reduzierung der endogenen Biotinkonzentration des Empfängers vor der, zusammen mit der oder im Anschluss an die Verabreichung des ersten Konjugats umfasst.

6. Verwendung nach irgendeinem der Ansprüche 1 bis 3, worin das erste Konjugat einen Liganden umfasst und das Wirkstoffkonjugat einen Antiliganden umfasst, wobei vorzugsweise der Ligand Biotin ist und der Antiligand Streptavidin ist.

7. Verwendung nach irgendeinem der Ansprüche 1 bis 6, worin die Targeting-Gruppierung ein monoklonaler Antikörper oder ein Antigen-erkennendes Fragment davon ist, welche mit einem Antigen reaktionsfähig ist, das von einem NR-LU-10-Antikörper erkannt wird.

8. Kit umfassend die folgenden pharmazeutischen Zusammensetzungen zur gleichzeitigen oder aufeinanderfolgenden Verabreichung an einen Säugetier-Empfänger von:
a) einer ersten Zusammensetzung umfassend ein erstes Konjugat umfassend eine Targeting-Gruppierung und ein Element eines Ligand/Antiligand-Bindungspaares und
b) einer zweiten Zusammensetzung umfassend ein Wirkstoffkonjugat umfassend einen Cytokin- oder Wachstumsfaktor-Wirkstoff ausgewählt aus der Gruppe bestehend aus Cytokinen, Interleukinen, transformierendem Wachstumsfaktor-beta, Tumor-Nekrose-Faktoren, Interferonen, koloniestimulierenden Faktoren, vaskulärem Permeabilitätsfaktor und Selektinen und ein Ligand/Antiligand-Bindungspaar-Element, wobei das Wirkstoffkonjugat-Bindungspaar-Element komplementär zu dem des ersten Konjugats ist, und
c) gegebenenfalls einer dritten Zusammensetzung umfassend ein Clearing-Mittel, das in der Lage ist, das zirkulierende erste Konjugat zu Ashwell-Rezeptoren auf Leberzellen zu leiten, wodurch die Menge an zirkulierendem erstem Konjugat vor der Verabreichung des zweiten Konjugats verringert wird.

9. Kit nach Anspruch 8, worin der Cytokin- oder Wachstumsfaktor-Wirkstoff in der zweiten Zusammensetzung ausgewählt ist aus der Gruppe bestehend aus Tumor-Nekrose-Faktor, Interleukin-2, transformierendem Wachstumsfaktor-beta, Interleukin-4, Interleukin-10, Interferon-gamma, Interferon-alpha, Granulozyten-Makrophagen-koloniestimulierendem Faktor und Granulozytenkoloniestimulierendem Faktor.

10. Kit nach Anspruch 8 oder 9, das ein oder mehrere weitere Merkmale aufweist, die in Anspruch 4 oder Anspruch 6 definiert sind, worin, wenn das erste Konjugat einen Antiliganden umfasst, dieser Streptavidin ist und der Ligand Biotin ist oder, wenn das erste Konjugat einen Liganden umfasst, dieser Biotin ist und der Antiligand Streptavidin ist.

## Revendications

1. Utilisation d'un conjugué d'agent actif qui comprend une cytokine ou un agent actif de facteur de croissance sélectionné dans le groupe constitué des cytokines, des interleukines, du facteur de croissance transformant beta, de facteurs de nécrose des tumeurs, des interférons, de facteurs de stimulation des colonies, du facteur de perméabilité vasculaire et des sélectines, et un élément d'une paire de liaison ligand/antitigand, dans la fabrication d'une composi ti on destinée à être utilisée dans un procédé d'augmentation de la localisation d'un agent actif fortement toxique sur le site d'une cellule cible chez un mammifère bénéficiaire, lequel procédé comprend les étapes qui consistent à:
administrer au bénéficiaire un premier conjugué qui comprend un fragment de ciblage et un élément d'une paire de liaison ligand/antiligand complémentaire de celle du conjugué d'agent actif et
ensuite, administrer le conjugué d'agent actif au receveur.

2. Utilisation selon la revendication 1, dans laquelle la cytokine ou l'agent actif de facteur de croissance est choisi dans le groupe constitué du facteur de nécrose des tumeurs, de l'interleukine-2, du facteur de croissance transformant beta, de l'interleukine-4, de l'interleukine-10, de l'interféron gamma, de l'interféron alpha, du facteur de stimulation des colonies de macrophages de granulocytes et du facteur de stimulation des colonies de granulocytes.

3. utilisation selon la revendication 1 ou 2, dans laquelle le procédé comprend en outre l'étape qui consiste à administrer au bénéficiaire un agent de nettoyage qui est en mesure de diriger le premier conjugué en circulation vers les récepteurs d'Ashwell des hépatocytes pour ainsi diminuer la quantité du premier conjugué en circulation avant d'administrer le conjugué d'agent actif.

4. Utilisation selon l'une quelconque des revendications précédentes, dans laquelle le premier conjugué comprend un antiligand et le conjugué d'agent actif comprend un ligand, l'antiligand étant de préférence la streptavidine et le ligand la biotine.

5. Utilisation selon la revendication 4, dans laquelle le ligand est la biotine et dans laquelle le procédé comprend en outre l'étape qui consiste à réduire le niveau endogène de biotine du bénéficiaire avant, pendant ou après l'administration du premier conjugué.

6. Utilisation selon l'une quelconque des revendications 1 à 3, dans laquelle le premier conjugué comprend un ligand et le conjugué d'agent actif comprend un antiligand, le ligand étant de préférence la biotine et l'antiligand la streptavidine.

7. utilisation selon l'une quelconque des revendications 1 à 6, dans laquelle la fraction de ciblage est un anticorps monoclonal ou un fragment de reconnaissance d'antigène de ce dernier, qui réagit avec un antigène reconnu par l'anticorps NR-LU-10.

8. Trousse comprenant les compositions pharmaceutiques ci-dessous pour l'administration conjointe ou successive à un mammifère bénéficiaire:
a) une première composition qui comprend un premier conjugué qui comprend une fraction de ciblage et un élément d'une paire de liaison ligand-antiligand,
b) une deuxième composition qui comprend un conjugué d'agent actif qui comprend une cytokine ou un agent actif sur le facteur de croissance, qui sont sélectionnés dans le groupe constitué des cytoki nes , des interleukines, du facteur de croissance transformant beta, des facteurs de nécrose des tumeurs, des interférons, des facteurs de stimulation des colonies, du facteur de perméabilité vasculaire et des sélectines ; et un élément d'une paire de liaison ligand/antiligand, l'élément de la paire de liaison au conjugué d'agent actif étant complémentaire de celui du premier conjugué et
c) facultativement, une troisième composition qui comprend un agent de nettoyage capable d'envoyer le premier conjugué en circulation vers les récepteurs d'Ashwell des hépatocytes pour ainsi diminuer la quantité de premier conjugué en circulation avant l'administration du deuxième conjugué.

9. Trousse selon la revendication 8, dans laquelle la cytokine ou l'agent actif sur le facteur de croissance de la deuxième composition est sélectionné dans le groupe constitué du facteur de nécrose des tumeurs, de l'interleukine-2, du facteur de croissance transformant beta, de l'interleukine-4, de l'interleukine-10, de l'interféron gamma, de l'interféron alpha, du facteur de stimulation des colonies de macrophage des granulocytes et du facteur de stimulation des colonies de granulocytes.

10. Trousse selon la revendication 8 ou 9, qui présente la ou les caractéristiques définies dans la revendication 4 ou la revendication 6, et dans laquelle, lorsque le premier conjugué comprend un antiligand, ce dernier est la streptavidine et le ligand est la biotine ou, lorsque le premier conjugué comprend un ligand, ce dernier est la biotine et l'antiligand est la streptavidine.
